# FASCICULE DE BREVET EUROPEEN

(11) **EP 2 697 302 B1**
(45) Date de publication et mention de la délivrance du brevet: **03.06.2015**
(21) Numéro de dépôt: 12715955.6
(22) Date de dépôt: 16.04.2012
(51) Int. Cl.: C08K 5/00, C08K 5/3492, C07D 253/07, C08L 21/00

(54) **COMPOSITION DE CAOUTCHOUC COMPRENANT UN DÉRIVÉ DE LA 1,2,4-TRIAZINE**
KAUTSCHUKZUSAMMENSETZUNG MIT EINEM 1,2,4-TRIAZINDERIVAT
RUBBER COMPOSITION INCLUDING A 1,2,4-TRIAZINE DERIVATIVE

(30) Priorité: 14.04.2011 FR 1101174
(43) Date de publication de la demande: 19.02.2014
(73) Titulaire: Compagnie Générale des Etablissements Michelin, 63000 Clermont-Ferrand (FR); MICHELIN Recherche et Technique S.A., 1763 Granges-Paccot (CH)
(72) Inventeur: VEYLAND, Anne, F-63040 Clermont-Ferrand Cedex 09 (FR); SEEBOTH, Nicolas, F-63040 Clermont-Ferrand Cedex 09 (FR)
(74) Mandataire: Sidhu, Alban
(86) Numéro de dépôt international: PCT/EP2012/056907
(87) Numéro de publication internationale: WO 2012/140255

(56) Documents cités:
- US-A- 3 938 574
- YOUNES MANSOUR I ET AL: "Synthesis of [1,2,4]triazolo[3',4':3,4] [1,2,4]triazino[5,6-b]indole derivatives and 3-substituted 5-ethyl-H-1,2,4-triazino[5,6-b] indoles", ARCHIV DER PHARMAZIE, WILEY - VCH VERLAG GMBH & CO. KGAA, DE, vol. 320, no. 12, 1 janvier 1987 (1987-01-01), pages 1196-1202, XP009115783, ISSN: 0365-6233

## Description

La présente invention se rapporte à une composition de caoutchouc utilisable notamment pour la fabrication de pneumatiques ou de produits semi-finis pour pneumatiques tels que des bandes de roulement, ladite composition étant à base d'un élastomère diénique, d'une charge renforçante et d'un système de vulcanisation comprenant un composé 1,2,4-triazine particulier.

La vulcanisation des élastomères diéniques par le soufre est largement utilisée dans l'industrie du caoutchouc, en particulier celle du pneumatique. Pour vulcaniser les élastomères diéniques, on utilise un système de vulcanisation relativement complexe comportant, en plus du soufre, un accélérateur primaire de vulcanisation, tels que les sulfénamides à noyau benzothiazole, ainsi que divers accélérateurs secondaires ou activateurs de vulcanisation, tout particulièrement des dérivés du zinc tels que l'oxyde de zinc (ZnO) seul ou utilisé avec des acides gras.

Les sulfénamides à noyau benzothiazole utilisés comme accélérateurs primaires de vulcanisation sont par exemple la N-cyclohexyl-2-benzothiazyle sulfénamide (en abrégé « CBS »), la N,N-dicyclohexyl-2-benzothiazyle sulfénamide (en abrégé « DCBS »), la N-ter-butyl-2-benzothiazyle sulfénamide (en abrégé « TBBS ») et les mélanges de ces composés.

A titre d'accélérateur primaire de vulcanisation, est également connu le 2-(1,3-benzothiazol-2-yldithio)-1,3-benzothiazole (en abrégé « MBTS »).

Les compositions de caoutchouc doivent présenter une réticulation suffisante tout en conservant un compromis acceptable entre les différentes propriétés rhéométriques.

En réponse à ce problème, la demanderesse a découvert une nouvelle composition de caoutchouc comprenant à titre d'accélérateur de vulcanisation un composé 1,2,4-triazine. Cette nouvelle composition de caoutchouc permet d'obtenir une composition vulcanisée à l'aide d'accélérateurs alternatifs aux accélérateurs connus, idéalement avec un compromis de propriétés rhéométriques similaire à celui obtenu avec des compositions de caoutchouc contenant des accélérateurs de vulcanisation classiquement utilisés.

L'invention a donc pour objet une composition de caoutchouc pour la fabrication de pneumatiques, à base d'un ou plusieurs élastomères diéniques, d'une ou plusieurs charges renforçantes et d'un système de vulcanisation, ledit système de vulcanisation comprenant un ou plusieurs composés 1,2,4-triazines choisis parmi les composés de formule 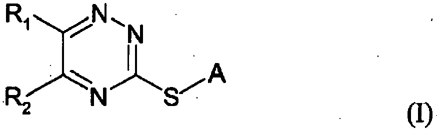 et 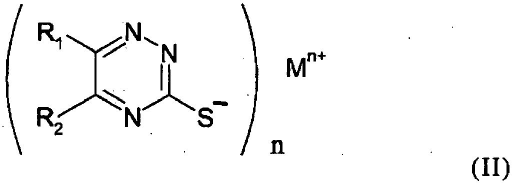 où
R₁ et R₂ représentent indépendamment H, un halogène, un groupe choisi par les groupes alcoxy et (C₁-C₁₀)alkylthio, ou un groupe hydrocarboné en C₁-C₂₅ choisi parmi les groupes alkyles linéaires, ramifiés ou cycliques, les groupes aralkyles, les groupes alkylaryles et les groupes aryles, et éventuellement interrompus par un ou plusieurs hétéroatomes, R₁ pouvant former un cycle avec R₂,
- A est choisi parmi
   - -H,
   - un groupe 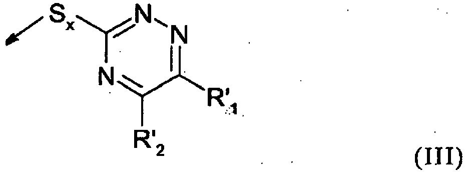 où
      R'₁ est identique à R₁,
      R'₂ est identique à R₂,
      x est un entier supérieur ou égal à 1, préférentiellement inférieur ou égal à 10, plus préférentiellement inférieur ou égal à 8 et très préférentiellement inférieur ou égal à 6,
   - un groupe 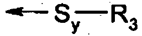 où R₃ représente un groupe hydrocarboné en C₁-C₁₈ éventuellement interrompu par un ou plusieurs hétéroatomes, de préférence un groupe CR'₃R₄R₅, où R'₃, R₄ et R₅ représentent indépendamment un groupe en C₁-C₁₇ éventuellement interrompu par un ou plusieurs hétéroatomes,
      y est un entier supérieur ou égal à 1, préférentiellement inférieur ou égal à 10, plus préférentiellement inférieur ou égal à 8 et très préférentiellement inférieur ou égal à 6,
   - un groupe 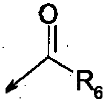 où R₆ est un groupe hydrocarboné en C₁-C₁₈, éventuellement interrompu par un ou plusieurs hétéroatomes,
   - un groupe 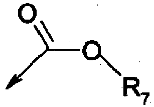 où R₇ est un groupe hydrocarboné en C₁-C₁₈, éventuellement interrompu par un ou plusieurs hétéroatomes,
   - un groupe 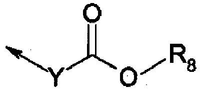 où Y est une chaîne hydrocarbonée en C₁-C₁₈, éventuellement interrompue ou substituée par un ou plusieurs hétéroatomes ; R₈ est un groupe hydrocarboné en C₁-C₁₈, éventuellement interrompu par un ou plusieurs hétéroatomes,
   - un groupe 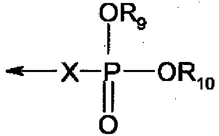 où X est une chaîne hydrocarbonée en C₁-C₁₈, éventuellement interrompue ou substituée par un ou plusieurs hétéroatomes ; R₉ et R₁₀ représentent indépendamment un groupe hydrocarboné en C₁-C₁₈, éventuellement interrompu par un ou plusieurs hétéroatomes, et
   - un groupe 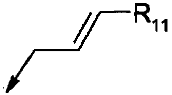 où R₁₁ est H ou un groupe hydrocarboné en C₁-C₁₈, éventuellement interrompu par un ou plusieurs hétéroatomes,
   Mⁿ⁺ représente un cation métallique ou non métallique,
   n représente un entier variant entre 1 et 4.

Dans les formules qui précèdent et qui suivent, la flèche (→) désigne le point d'attachement des groupes A et Aₐ sur l'atome de soufre respectivement des formules (I) et (I').

L'invention a également pour objet un procédé pour préparer une composition de caoutchouc pour la fabrication de pneumatiques selon l'invention, comprenant les étapes suivantes:
- incorporer à ou auxdits élastomères diéniques, au cours d'une première étape, la ou les charges renforçantes, en malaxant thermomécaniquement le tout, en une ou plusieurs fois, jusqu'à atteindre une température maximale comprise entre 110°C et 190°C ;
- incorporer ensuite, au cours d'une seconde étape, le système de réticulation et malaxer le tout jusqu'à une température maximale inférieure à 110°C.

De préférence, l'invention concerne le procédé tel que défini ci-dessus, dans lequel entre le malaxage thermomécanique et l'incorporation du système de réticulation, on procède au refroidissement de l'ensemble à une température inférieure ou égale à 100°C. De préférence dans ce cas, la dernière étape est réalisée sur un deuxième mélangeur.

L'invention a encore pour objet l'utilisation d'une composition selon l'invention pour la fabrication d'un article fini ou d'un produit semi-fini destiné à un système de liaison au sol de véhicule automobile, tel que pneumatique, appui interne de sécurité pour pneumatique, roue, ressort en caoutchouc, articulation élastomérique, autre élément de suspension et anti-vibratoire. En particulier, la composition selon l'invention peut être utilisée pour la fabrication de produits semi-finis en caoutchouc destinés à des pneumatiques, tel que les bandes de roulement, les nappes d'armature de sommet, les flancs, les nappes d'armature de carcasse, les bourrelets, les protecteurs, les sous-couches, les blocs de caoutchouc et autres gommes internes, notamment les gommes de découplage, destinés à assurer la liaison ou l'interface entre les zones précitées des pneumatiques.

L'invention a encore pour objet un article fini ou produit semi-fini destiné à un système de liaison au sol de véhicule automobile, en particulier les pneumatiques et produits semi-finis pour pneumatiques, en particulier les bandes de roulement, comprenant une composition selon l'invention. Les pneumatiques conformes à l'invention sont notamment destinés à des véhicules tourisme, des deux-roues, comme à des véhicules industriels choisis parmi camionnettes, « Poids-lourd »- i.e. métro, bus, engins de transport routier (camions, tracteurs, remorques), véhicules hors-la-route -, engin agricoles ou de génie civil, avions, autres véhicules de transport ou de manutention.

L'invention a encore pour objet l'utilisation comme accélérateur de vulcanisation dans une composition à base d'un ou plusieurs élastomères diéniques, d'une ou plusieurs charges renforçantes et d'un système de vulcanisation, d'un ou plusieurs composés 1,2,4-triazines de formule (I) et/ou (II).

L'invention a enfin pour objet un 1,2,4-triazine de formule 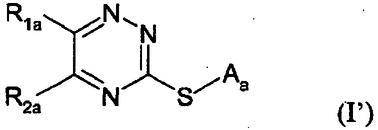 où
R₁ₐ et R₂ₐ représentent indépendamment H, un halogène, un groupe choisi par les groupes alcoxy et (C₁-C₁₀)alkylthio, ou un groupe hydrocarboné en C₁-C₂₅ choisi parmi les groupes alkyles linéaires, ramifiés ou cycliques, les groupes aralkyles, les groupes alkylaryles et les groupes aryles, et éventuellement interrompus par un ou plusieurs hétéroatomes, R₁ₐ pouvant former un cycle avec R₂ₐ,
- Aₐ est choisi parmi
   - un groupe 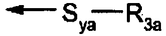 où R₃ₐ représente un groupe hydrocarboné en C₁-C₁₈, éventuellement interrompu par un ou plusieurs hétéroatomes, à la condition que R₃ₐ ne comporte pas de 1,2,4-triazine, et à la condition que le composé (I') ne soit pas de formule 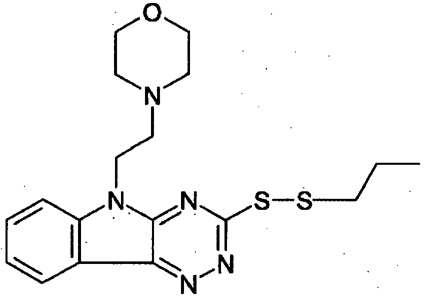 yₐ est un entier supérieur ou égal à 1, préférentiellement inférieur ou égal à 10, plus préférentiellement inférieur ou égal à 8 et très préférentiellement inférieur ou égal à 6,
   - un groupe 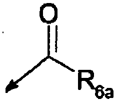 où R₆ₐ est un groupe hydrocarboné en C₁-C₁₈, éventuellement interrompu par un ou plusieurs hétéroatomes, étant entendu que R₆ₐ est différent d'un groupe phényle éventuellement substitué, et étant entendu que si R₆ₐ représente un groupe méthyle, R₁ₐ et R₂ₐ ne forment pas de cycle et R₁ₐ et R₂ₐ ne représentent pas respectivement un méthyle et un hydroxyle,
   - un groupe 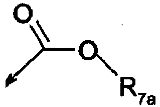 où R₇ₐ est un groupe hydrocarboné en C₁-C₁₈, éventuellement interrompu par un ou plusieurs hétéroatomes, étant entendu que si R₇ₐ représente un groupe éthyle, et R₂ₐ ne forment pas de cycle,
   - un groupe 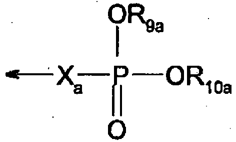 où Xₐ est une chaîne hydrocarbonée en C₁-C₁₈, éventuellement interrompue ou substituée par un ou plusieurs hétéroatomes ;
   R₉ₐ et R₁₀ₐ représentent indépendamment un groupe hydrocarboné en C₁-C₁₈, éventuellement interrompu par un ou plusieurs hétéroatomes.

L'invention ainsi que ses avantages seront aisément compris à la lumière de la description et des exemples de réalisation qui suivent.

### I. Mesures et tests utilisés

Les composés synthétisés comme accélérateurs de vulcanisation ont été caractérisés par spectrométrie de masse et par RMN avec les appareils et méthodes indiqués ci-après.

### Spectrométrie de masse

L'analyse structurale du produit attendu ainsi que la détermination des structures des impuretés ont été réalisées par analyse ID/IC (introduction directe à la spectrométrie de masse avec l'ionisation chimique comme mode d'ionisation).

Conditions opératoires :
Gaz réactant : méthane / ammoniac (85/15) à 2ml/min
Température de la source : 200°C
Gamme de masse balayée : 50 à 1000m/z

### RMN

L'analyse structurale ainsi que la détermination des puretés molaires des molécules de synthèses sont réalisées par une analyse RMN. Les spectres sont acquis sur un spectromètre Avance 500 MHz BRUKER équipé d'une sonde " large bande " BBIz-grad 5 mm. L'expérience RMN 1H quantitative, utilise une séquence simple impulsion 30° et un délai de répétition de 3 secondes entre chacune des 64 acquisitions. Le spectre RMN 1H couplé aux expériences 2D HSQC 1H/13C et HMBC 1H/13C permettent la détermination structurale des molécules (cf tableaux d'attributions). Les quantifications molaires sont réalisées à partir du spectre RMN 1D 1H quantitatif.

Les compositions de caoutchouc, dans lesquelles sont testés les accélérateurs de vulcanisation 1,2,4-triazines, sont caractérisées, avant et après cuisson, comme indiqué ci-après.

### Rhéom étrie

Les mesures sont effectuées à 150°C avec un rhéomètre à chambre oscillante, selon la norme DIN 53529 - partie 3 (juin 1983). L'évolution du couple rhéométrique, ΔCouple (en dN.m), en fonction du temps décrit l'évolution de la rigidification de la composition par suite de la réaction de vulcanisation. Les mesures sont traitées selon la norme DIN 53529 - partie 2 (mars 1983) (à l'exception de la constante de vitesse de conversion) : T₀ est le délai d'induction, c'est-à-dire le temps nécessaire au début de la réaction de vulcanisation ; T_{α} (par exemple T₉₉) est le temps nécessaire pour atteindre une conversion de α%, c'est-à-dire α% (par exemple 99%) de l'écart entre les couples minimum et maximum.

On mesure également la constante de vitesse de conversion notée K (exprimée en min⁻¹), d'ordre 1, calculée entre 30% et 80% de conversion, qui permet d'apprécier la cinétique de vulcanisation. Cette constante K est déterminée en mesurant la pente de la montée en couple sur le rhéogramme de cuisson à 150°C.

### II. Conditions de réalisation de l'invention

Comme expliqué précédemment, la composition selon l'invention est à base d'un ou plusieurs élastomères diéniques, d'une ou plusieurs charges renforçantes et d'un système de vulcanisation.

Par l'expression composition « à base de », il faut entendre une composition comportant le mélange et/ou le produit de réaction des différents constituants utilisés, certains de ces constituants de base étant susceptibles de, ou destinés à réagir entre eux, au moins en partie, lors des différentes phases de fabrication de la composition, en particulier au cours de sa vulcanisation.

Dans la présente description, sauf indication expresse différente, tous les pourcentages (%) sont des % en masse. D'autre part, tout intervalle de valeurs désigné par l'expression "entre a et b" représente le domaine de valeurs allant de plus de a à moins de b (c'est-à-dire bornes a et b exclues) tandis que tout intervalle de valeurs désigné par l'expression "de a à b" signifie le domaine de valeurs allant de a jusqu'à b (c'est-à-dire incluant les bornes strictes a et b).

### II-1. Elastomère diénique

Par élastomère ou caoutchouc "diénique", doit être compris de manière connue un élastomère issu au moins en partie (i.e., un homopolymère ou un copolymère) de monomères diènes (monomères porteurs de deux doubles liaisons carbone-carbone, conjuguées ou non).

Ces élastomères diéniques peuvent être classés dans deux catégories : "essentiellement insaturés" ou "essentiellement saturés". On entend en général par "essentiellement insaturé", un élastomère diénique issu au moins en partie de monomères diènes conjugués, ayant un taux de motifs ou unités d'origine diénique (diènes conjugués) qui est supérieur à 15% (% en moles) ; c'est ainsi que des élastomères diéniques tels que les caoutchoucs butyle ou les copolymères de diènes et d'alpha-oléfines type EPDM n'entrent pas dans la définition précédente et peuvent être notamment qualifiés d'élastomères diéniques "essentiellement saturés" (taux de motifs d'origine diénique faible ou très faible, toujours inférieur à 15%). Dans la catégorie des élastomères diéniques "essentiellement insaturés", on entend en particulier par élastomère diénique "fortement insaturé" un élastomère diénique ayant un taux de motifs d'origine diénique (diènes conjugués) qui est supérieur à 50%.

Ces définitions étant données, on entend plus particulièrement par élastomère diénique susceptible d'être utilisé dans les compositions conformes à l'invention :
(a) - tout homopolymère obtenu par polymérisation d'un monomère diène conjugué ayant de 4 à 12 atomes de carbone ;
(b) - tout copolymère obtenu par copolymérisation d'un ou plusieurs diènes conjugués entre eux ou avec un ou plusieurs composés vinyle aromatique ayant de 8 à 20 atomes de carbone;
(c) - un copolymère ternaire obtenu par copolymérisation d'éthylène, d'une α-oléfine ayant 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène ;
(d) - un copolymère d'isobutène et d'isoprène (caoutchouc butyle), ainsi que les versions halogénées, en particulier chlorées ou bromées, de ce type de copolymère.

Bien qu'elle s'applique à tout type d'élastomère diénique, l'homme du métier du pneumatique comprendra que la présente invention est de préférence mise en oeuvre avec des élastomères diéniques essentiellement insaturés, en particulier du type (a) ou (b) ci-dessus.

A titre de diènes conjugués conviennent notamment le butadiène-1,3, le 2-méthyl-1,3-butadiène, les 2,3-di(alkyle en C₁-C₅)-1,3-butadiènes tels que par exemple le 2,3-diméthyl-1,3-butadiène, le 2,3-diéthyl-1,3-butadiène, le 2-méthyl-3-éthyl-1,3-butadiène, le 2-méthyl-3-isopropyl-1,3-butadiène, un aryl-1,3-butadiène, le 1,3-pentadiène, le 2,4-hexadiène. A titre de composés vinylaromatique conviennent par exemple le styrène, l'ortho-, méta-, para-méthylstyrène, le mélange commercial "vinyle-toluène", le para-tertiobutylstyrène, les méthoxystyrènes, les chlorostyrènes, le vinylmésitylène, le divinylbenzène, le vinylnaphtalène.

Les copolymères peuvent contenir entre 99% et 20% en poids d'unités diéniques et entre 1% et 80% en poids d'unités vinylaromatiques. Les élastomères peuvent avoir toute microstructure qui est fonction des conditions de polymérisation utilisées, notamment de la présence ou non d'un agent modifiant et/ou randomisant et des quantités d'agent modifiant et/ou randomisant employées. Les élastomères peuvent être par exemple à blocs, statistiques, séquencés, microséquencés, et être préparés en dispersion, en émulsion ou en solution ; ils peuvent être couplés et/ou étoilés ou encore fonctionnalisés avec un agent de couplage et/ou d'étoilage ou de fonctionnalisation. Pour un couplage à du noir de carbone, on peut citer par exemple des groupes fonctionnels comprenant une liaison C-Sn ou des groupes fonctionnels aminés tels que aminobenzophénone par exemple ; pour un couplage à une charge inorganique renforçante telle que silice, on peut citer par exemple des groupes fonctionnels silanol ou polysiloxane ayant une extrémité silanol (tels que décrits par exemple dans FR 2 740 778,US 6 013 718 ou WO 2008/141702), des groupes alkoxysilane (tels que décrits par exemple dans FR 2 765 882 ou US 5 977 238), des groupes carboxyliques (tels que décrits par exemple dans WO 01/92402 ou US 6 815 473, WO 2004/096865 ou US 2006/0089445) ou encore des groupes polyéthers (tels que décrits par exemple dans EP 1 127 909, US 6 503 973 WO 2009/000750 ou WO 2009/000752). Comme autres exemples d'élastomères fonctionnalisés, on peut citer également des élastomères (tels que SBR, BR, NR ou IR) du type époxydé.

Conviennent les polybutadiènes et en particulier ceux ayant une teneur (% molaire) en unités -1,2 comprise entre 4% et 80% ou ceux ayant une teneur (% molaire) en cis-1,4 supérieure à 80%, les polyisoprènes, les copolymères de butadiène-styrène et en particulier ceux ayant une Tg (température de transition vitreuse, mesurée selon ASTM D3418) comprise entre 0°C et - 70°C et plus particulièrement entre - 10°C et - 60°C, une teneur en styrène comprise entre 5% et 60% en poids et plus particulièrement entre 20% et 50%, une teneur (% molaire) en liaisons -1,2 de la partie butadiénique comprise entre 4% et 75%, une teneur (% molaire) en liaisons trans-1,4 comprise entre 10% et 80%, les copolymères de butadiène-isoprène et notamment ceux ayant une teneur en isoprène comprise entre 5% et 90% en poids et une Tg de - 40°C à - 80°C, les copolymères isoprène-styrène et notamment ceux ayant une teneur en styrène comprise entre 5% et 50% en poids et une Tg comprise entre 5°C et - 50°C. Dans le cas des copolymères de butadiène-styrène-isoprène conviennent notamment ceux ayant une teneur en styrène comprise entre 5% et 50% en poids et plus particulièrement comprise entre 10% et 40%, une teneur en isoprène comprise entre 15% et 60% en poids et plus particulièrement entre 20% et 50%, une teneur en butadiène comprise entre 5% et 50% en poids et plus particulièrement comprise entre 20% et 40%, une teneur (% molaire) en unités -1,2 de la partie butadiénique comprise entre 4% et 85%, une teneur (% molaire) en unités trans -1,4 de la partie butadiénique comprise entre 6% et 80%, une teneur (% molaire) en unités -1,2 plus -3,4 de la partie isoprénique comprise entre 5% et 70% et une teneur (% molaire) en unités trans -1,4 de la partie isoprénique comprise entre 10% et 50%, et plus généralement tout copolymère butadiène-styrène-isoprène ayant une Tg comprise entre - 5°C et - 70°C.

En résumé, le ou les élastomères diéniques de la composition selon l'invention sont choisis préférentiellement dans le groupe des élastomères diéniques fortement insaturés constitué par les polybutadiènes (en abrégé "BR"), les polyisoprènes (IR) de synthèse, le caoutchouc naturel (NR), les copolymères de butadiène, les copolymères d'isoprène et les mélanges de ces élastomères. De tels copolymères sont plus préférentiellement choisis dans le groupe constitué par les copolymères de butadiène-styrène (SBR), les copolymères d'isoprène-butadiène (BIR), les copolymères d'isoprène-styrène (SIR) et les copolymères d'isoprène-butadiène-styrène (SBIR).

Selon un mode de réalisation, l'élastomère diénique est le caoutchouc naturel.

Selon un autre mode de réalisation particulier, l'élastomère diénique est majoritairement (i.e., pour plus de 50 pce) un SBR, qu'il s'agisse d'un SBR préparé en émulsion ("ESBR") ou d'un SBR préparé en solution ("SSBR"), ou un coupage (mélange) SBR/BR, SBR/NR (ou SBR/IR), BR/NR (ou BR/IR), ou encore SBR/BR/NR (ou SBR/BR/IR). Dans le cas d'un élastomère SBR (ESBR ou SSBR), on utilise notamment un SBR ayant une teneur en styrène moyenne, par exemple comprise entre 20% et 35% en poids, ou une teneur en styrène élevée, par exemple de 35 à 45%, une teneur en liaisons vinyliques de la partie butadiénique comprise entre 15% et 70%, une teneur (% molaire) en liaisons trans-1,4 comprise entre 15% et 75% et une Tg comprise entre - 10°C et - 55°C ; un tel SBR peut être avantageusement utilisé en mélange avec un BR possédant de préférence plus de 90% (% molaire) de liaisons cis-1,4.

Selon un autre mode de réalisation particulier, l'élastomère diénique est majoritairement (pour plus de 50 pce) un élastomère isoprénique. C'est le cas en particulier lorsque les compositions de l'invention sont destinées à constituer, dans les pneumatiques, les matrices de caoutchouc de certaines bandes de roulement (par exemple pour véhicules industriels), de nappes d'armature de sommet (par exemple de nappes de travail, nappes de protection ou nappes de frettage), de nappes d'armature de carcasse, de flancs, de bourrelets, de protecteurs, de sous-couches, de blocs de caoutchouc et autres gommes internes assurant l'interface entre les zones précitées des pneumatiques.

Par "élastomère isoprénique", on entend de manière connue un homopolymère ou un copolymère d'isoprène, en d'autres termes un élastomère diénique choisi dans le groupe constitué par le caoutchouc naturel (NR), les polyisoprènes de synthèse (IR), les différents copolymères d'isoprène et les mélanges de ces élastomères. Parmi les copolymères d'isoprène, on citera en particulier les copolymères d'isobutène-isoprène (caoutchouc butyle - IIR), d'isoprène-styrène (SIR), d'isoprène-butadiène (BIR) ou d'isoprène-butadiène-styrène (SBIR). Cet élastomère isoprénique est de préférence du caoutchouc naturel ou un polyisoprène cis-1,4 de synthèse; parmi ces polyisoprènes de synthèse, sont utilisés de préférence des polyisoprènes ayant un taux (% molaire) de liaisons cis-1,4 supérieur à 90%, plus préférentiellement encore supérieur à 98%.

Selon un autre mode de réalisation particulier, notamment lorsqu'elle est destinée à un flanc de pneumatique, à une gomme intérieure étanche de pneumatique sans chambre (ou autre élément imperméable à l'air), la composition conforme à l'invention peut contenir au moins un élastomère diénique essentiellement saturé, en particulier au moins un copolymère EPDM ou un caoutchouc butyle (éventuellement chloré ou bromé), que ces copolymères soient utilisés seuls ou en mélange avec des élastomères diéniques fortement insaturés tels que cités précédemment, notamment NR ou IR, BR ou SBR.

Selon un autre mode préférentiel de réalisation de l'invention, la composition de caoutchouc comprend un coupage d'un (un ou plusieurs) élastomère diénique dit "à haute Tg" présentant une Tg comprise entre - 70°C et 0°C et d'un (un ou plusieurs) élastomère diénique dit "à basse Tg" comprise entre -110°C et -80°C, plus préférentiellement entre -105°C et -90°C. L'élastomère à haute Tg est choisi de préférence dans le groupe constitué par les S-SBR, les E-SBR, le caoutchouc naturel, les polyisoprènes de synthèse (présentant un taux (% molaire) d'enchaînements cis-1,4 de préférence supérieur à 95%), les BIR, les SIR, les SBIR, et les mélanges de ces élastomères. L'élastomère à basse Tg comprend de préférence des unités butadiène selon un taux (% molaire) au moins égal à 70% ; il consiste de préférence en un polybutadiène (BR) présentant un taux (% molaire) d'enchaînements cis-1,4 supérieur à 90%.

Selon un autre mode particulier de réalisation de l'invention, la composition de caoutchouc comprend par exemple de 30 à 100 pce, en particulier de 50 à 100 pce, d'un élastomère à haute Tg en coupage avec 0 à 70 pce, en particulier de 0 à 50 pce, d'un élastomère à basse Tg ; selon un autre exemple, elle comporte pour la totalité des 100 pce un ou plusieurs SBR préparé(s) en solution.

Selon un autre mode particulier de réalisation de l'invention, l'élastomère diénique de la composition selon l'invention comprend un coupage d'un BR (à titre d'élastomère basse Tg) présentant un taux (% molaire) d'enchaînements cis-1,4 supérieur à 90%, avec un ou plusieurs S-SBR ou E-SBR (à titre d'élastomère(s) haute Tg).

La composition selon l'invention peut contenir un seul élastomère diénique ou un mélange de plusieurs élastomères diéniques, le ou les élastomères diéniques pouvant être utilisés en association avec tout type d'élastomère synthétique autre que diénique, voire avec des polymères autres que des élastomères, par exemple des polymères thermoplastiques.

### II-2. Charge renforçante

On peut utiliser tout type de charge renforçante connue pour ses capacités à renforcer une composition de caoutchouc utilisable pour la fabrication de pneumatiques, par exemple une charge organique tel que du noir de carbone, une charge inorganique renforçante telle que de la silice, ou encore un coupage de ces deux types de charge, notamment un coupage de noir de carbone et de silice.

Comme noirs de carbone conviennent tous les noirs de carbone, notamment les noirs du type HAF, ISAF, SAF conventionnellement utilisés dans les pneumatiques (noirs dits de grade pneumatique). Parmi ces derniers, on citera plus particulièrement les noirs de carbone renforçants des séries 100, 200 ou 300 (grades ASTM), comme par exemple les noirs N115, N134, N234, N326, N330, N339, N347, N375, ou encore, selon les applications visées, les noirs de séries plus élevées (par exemple N660, N683, N772). Les noirs de carbone pourraient être par exemple déjà incorporés à l'élastomère isoprénique sous la forme d'un masterbatch (voir par exemple demandes WO 97/36724 ou WO 99/16600).

Comme exemples de charges organiques autres que des noirs de carbone, on peut citer les charges organiques de polyvinylaromatique fonctionnalisé telles que décrites dans les demandes WO-A-2006/069792 et WO-A-2006/069793.

Par "charge inorganique renforçante", doit être entendu dans la présente demande, par définition, toute charge inorganique ou minérale (quelles que soient sa couleur et son origine (naturelle ou de synthèse), encore appelée charge "blanche", charge "claire" voire "charge non noire" ("non-black filler") par opposition au noir de carbone, capable de renforcer à elle seule, sans autre moyen qu'un agent de couplage intermédiaire, une composition de caoutchouc destinée à la fabrication de pneumatiques, en d'autres termes apte à remplacer, dans sa fonction de renforcement, un noir de carbone conventionnel de grade pneumatique ; une telle charge se caractérise généralement, de manière connue, par la présence de groupes hydroxyle (-OH) à sa surface.

L'état physique sous lequel se présente la charge inorganique renforçante est indifférent, que ce soit sous forme de poudre, de microperles, de granulés, de billes ou toute autre forme densifiée appropriée. Bien entendu on entend également par charge inorganique renforçante des mélanges de différentes charges inorganiques renforçantes, en particulier de charges siliceuses et/ou alumineuses hautement dispersibles telles que décrites ci-après.

Comme charges inorganiques renforçantes conviennent notamment des charges minérales du type siliceuse, en particulier de la silice (SiO₂), ou du type alumineuse, en particulier de l'alumine (Al₂O₃). La silice utilisée peut être toute silice renforçante connue de l'homme du métier, notamment toute silice précipitée ou pyrogénée présentant une surface BET ainsi qu'une surface spécifique CTAB toutes deux inférieures à 450 m²/g, de préférence de 30 à 400 m²/g. A titres de silices précipitées hautement dispersibles (dites "HDS"), on citera par exemple les silices Ultrasil 7000 et Ultrasil 7005 de la société Degussa, les silices Zeosil 1165MP, 1135MP et 1115MP de la société Rhodia, la silice Hi-Sil EZ150G de la société PPG, les silices Zeopol 8715, 8745 et 8755 de la Société Huber, les silices à haute surface spécifique telles que décrites dans la demande WO 03/16837.

Lorsque la composition selon l'invention est destinée à des bandes de roulement de pneumatique à faible résistance au roulement, la charge inorganique renforçante utilisée, en particulier s'il s'agit de silice, a de préférence une surface BET comprise entre 45 et 400 m²/g, plus préférentiellement comprise entre 60 et 300 m²/g.

De manière préférentielle, le taux de charge renforçante totale (noir de carbone et/ou charge inorganique renforçante telle que silice) est compris entre 20 et 200 pce, plus préférentiellement entre 30 et 150 pce, l'optimum étant de manière connue différent selon les applications particulières visées : le niveau de renforcement attendu sur un pneumatique vélo, par exemple, est bien sûr inférieur à celui exigé sur un pneumatique apte à rouler à grande vitesse de manière soutenue, par exemple un pneu moto, un pneu pour véhicule de tourisme ou pour véhicule utilitaire tel que Poids lourd.

Selon un mode de réalisation de l'invention, on utilise une charge renforçante comportant entre 30 et 150 pce, plus préférentiellement entre 50 et 120 pce de charge inorganique, particulièrement de silice, et optionnellement du noir de carbone ; le noir de carbone, lorsqu'il est présent, est utilisé de préférence à un taux inférieur à 20 pce, plus préférentiellement inférieur à 10 pce (par exemple entre 0,1 et 10 pce).

Selon un autre mode de réalisation, la charge renforçante est le noir de carbone.

Pour coupler la charge inorganique renforçante à l'élastomère diénique, on utilise de manière connue un agent de couplage (ou agent de liaison) au moins bifonctionnel destiné à assurer une connexion suffisante, de nature chimique et/ou physique, entre la charge inorganique (surface de ses particules) et l'élastomère diénique, en particulier des organosilanes ou des polyorganosiloxanes bifonctionnels.

On utilise notamment des silanes polysulfurés, dits "symétriques" ou "asymétriques" selon leur structure particulière, tels que décrits par exemple dans les demandes WO03/002648 (ou US 2005/016651) et WO03/002649 (ou US 2005/016650).

Conviennent en particulier, sans que la définition ci-après soit limitative, des silanes polysulfurés dits "symétriques" répondant à la formule générale (III) suivante:

(III) Z - D - Sₚ - D - Z

dans laquelle:
- p est un entier de 2 à 8 (de préférence de 2 à 5) ;
- D est un radical hydrocarboné divalent (de préférence des groupements alkylène en C₁-C₁₈ ou des groupements arylène en C₆-C₁₂, plus particulièrement des alkylènes en C₁-C₁₀, notamment en C₁-C₄, en particulier le propylène) ;
- Z répond à l'une des formules ci-après: 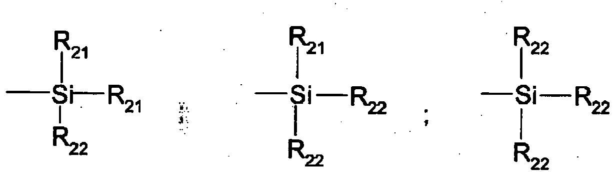 dans lesquelles:
   - les radicaux R₂₁, substitués ou non substitués, identiques ou différents entre eux, représentent un groupe alkyle en C₁-C₁₈, cycloalkyle en C₅-C₁₈ ou aryle en C₆-C₁₈ (de préférence des groupes alkyle en C₁-C₆, cyclohexyle ou phényle, notamment des groupes alkyle en C₁-C₄, plus particulièrement le méthyle et/ou l'éthyle).
   - les radicaux R₂₂, substitués ou non substitués, identiques ou différents entre eux, représentent un groupe alkoxyle en C₁-C₁₈ ou cycloalkoxyle en C₅-C₁₈ (de préférence un groupe choisi parmi alkoxyles en C₁-C₈ et cycloalkoxyles en C₅-C₈, plus préférentiellement encore un groupe choisi parmi alkoxyles en C₁-C₄, en particulier méthoxyle et éthoxyle).

Dans le cas d'un mélange d'alkoxysilanes polysulfurés répondant à la formule (III) ci-dessus, notamment des mélanges usuels disponibles commercialement, la valeur moyenne des "p" est un nombre fractionnaire de préférence compris entre 2 et 5, plus préférentiellement proche de 4. Mais l'invention peut être aussi avantageusement mise en oeuvre par exemple avec des alkoxysilanes di sulfurés (p = 2).

A titre d'exemples de silanes polysulfurés, on citera plus particulièrement les polysulfures (notamment disulfures, trisulfures ou tétrasulfures) de bis-(alkoxyl(C₁-C₄)-alkyl(C₁-C₄)silyl-alkyl(C₁-C₄)), comme par exemple les polysulfures de bis(3-triméthoxysilylpropyl) ou de bis(3-triéthoxysilylpropyl). Parmi ces composés, on utilise en particulier le tétrasulfure de bis(3-triéthoxysilylpropyl), en abrégé TESPT, de formule [(C₂H₅O)₃Si(CH₂)₃S₂]₂ ou le disulfure de bis-(triéthoxysilylpropyle), en abrégé TESPD, de formule [(C₂H₅O)₃Si(CH₂)₃S]₂. On citera également à titre d'exemples préférentiels les polysulfures (notamment disulfures, trisulfures ou tétrasulfures) de bis-(monoalkoxyl(C₁-C₄)-dialkyl(C₁-C₄)silylpropyl), plus particulièrement le tétrasulfure de bis-monoéthoxydiméthylsilylpropyl tel que décrit dans la demande de brevet WO 02/083782 (ou US 2004/132880).

A titre d'agent de couplage autre qu'alkoxysilane polysulfuré, on citera notamment des POS (polyorganosiloxanes) bifonctionnels ou encore des polysulfures d'hydroxysilane (R₂₂ = OH dans la formule III ci-dessus) tels que décrits dans les demandes de brevet WO 02/30939 (ou US 6,774,255) et WO 02/31041 (ou US 2004/051210), ou encore des silanes ou POS porteurs de groupements fonctionnels azo-dicarbonyle, tels que décrits par exemple dans les demandes de brevet WO 2006/125532, WO 2006/125533, WO 2006/125534.

Dans les compositions de caoutchouc conformes à l'invention, la teneur en agent de couplage est préférentiellement comprise entre 4 et 12 pce, plus préférentiellement entre 3 et 8 pce.

L'homme du métier comprendra qu'à titre de charge équivalente de la charge inorganique renforçante décrite dans le présent paragraphe, pourrait être utilisée une charge renforçante d'une autre nature, notamment organique, dès lors que cette charge renforçante serait recouverte d'une couche inorganique telle que silice, ou bien comporterait à sa surface des sites fonctionnels, notamment hydroxyles, nécessitant l'utilisation d'un agent de couplage pour établir la liaison entre la charge et l'élastomère.

### II.3 Système de vulcanisation

Le système de vulcanisation proprement dit est à base de soufre (ou d'un agent donneur de soufre) et d'un accélérateur primaire de vulcanisation. A ce système de vulcanisation de base viennent s'ajouter, incorporés au cours de la première phase non-productive et/ou au cours de la phase productive telles que décrites ultérieurement, divers accélérateurs secondaires ou activateurs de vulcanisation connus tels qu'oxyde de zinc, acide stéarique ou composés équivalents, dérivés guanidiques (en particulier diphénylguanidine).

Le soufre est utilisé à un taux préférentiel compris entre 0,5 et 10 pce, plus préférentiellement compris entre 0,5 et 5 pce, en particulier entre 0,5 et 3 pce lorsque la composition de l'invention est destinée, selon un mode préférentiel de l'invention, à constituer une bande de roulement de pneumatique.

L'accélérateur primaire de vulcanisation doit permettre une réticulation des compositions de caoutchouc dans des temps industriellement acceptables, tout en préservant un délai minimum de sécurité (« temps de grillage ») au cours duquel les compositions peuvent être mises en forme sans risque de vulcanisation prématurée (« grillage »).

Selon l'invention, le système de vulcanisation comprend, à titre d'accélérateur primaire de vulcanisation, un ou plusieurs composés 1,2,4-triazines choisis parmi les composés de formule 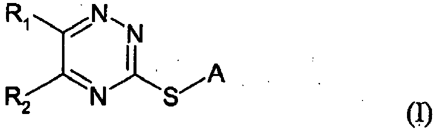 et 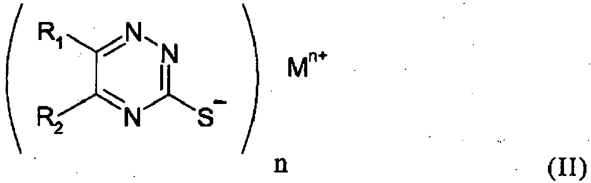 où
R₁ et R₂ représentent indépendamment H, un halogène, un groupe choisi par les groupes alcoxy et (C₁-C₁₀)alkylthio, ou un groupe hydrocarboné en C₁-C₂₅ choisi parmi les groupes alkyles linéaires, ramifiés ou cycliques, les groupes aralkyles, les groupes alkylaryles et les groupes aryles, et éventuellement interrompus par un ou plusieurs hétéroatomes, R₁ pouvant former un cycle avec R₂,
- A est choisi parmi
   - -H,
   - un groupe 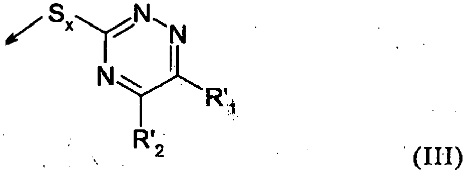 où
      R'₁ est identique à R₁,
      R'₂ est identique à R₂,
      x est un entier supérieur ou égal à 1, préférentiellement inférieur ou égal à 10, plus préférentiellement inférieur ou égal à 8 et très préférentiellement inférieur ou égal à 6,
   - un groupe 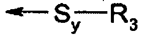 où R₃ représente un groupe hydrocarboné en C₁-C₁₈ éventuellement interrompu par un ou plusieurs hétéroatomes, de préférence un groupe CR'₃R₄R₅, où R'₃, R₄ et R₅ représentent indépendamment un groupe en C₁-C₁₇ éventuellement interrompu par un ou plusieurs hétéroatomes,
      y est un entier supérieur ou égal à 1, préférentiellement inférieur ou égal à 10, plus préférentiellement inférieur ou égal à 8 et très préférentiellement inférieur ou égal à 6,
   - un groupe 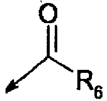 où R₆ est un groupe hydrocarboné en C₁-C₁₈, éventuellement interrompu par un ou plusieurs hétéroatomes,
   - un groupe 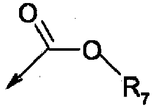 où R₇ est un groupe hydrocarboné en C₁-C₁₈, éventuellement interrompu par un ou plusieurs hétéroatomes,
   - un groupe 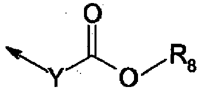 où Y est une chaîne hydrocarbonée en C₁-C₁₈, éventuellement interrompue ou substituée par un ou plusieurs hétéroatomes ;
      R₈ est un groupe hydrocarboné en C₁-C₁₈, éventuellement interrompu par un ou plusieurs hétéroatomes,
   - un groupe 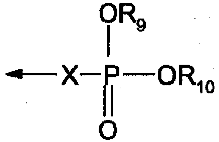 où X est une chaîne hydrocarbonée en C₁-C₁₈, éventuellement interrompue ou substituée par un ou plusieurs hétéroatomes ;
      R₉ et Rio représentent indépendamment un groupe hydrocarboné en C₁-C₁₈, éventuellement interrompu par un ou plusieurs hétéroatomes, et
   - un groupe 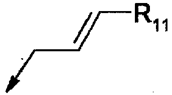 où R₁₁ est H ou un groupe hydrocarboné en C₁-C₁₈, éventuellement interrompu par un ou plusieurs hétéroatomes,
   Mⁿ⁺ représente un cation métallique ou non métallique,
   n représente un entier variant entre 1 et 4.

Les composés de formule (I) ou (II) peuvent avantageusement remplacer en tout ou partie les composés accélérateurs classiquement utilisés.

Par groupe alkyle cyclique, on entend un groupe alkyle constitué d'un ou plusieurs cycles.

Par groupe ou chaîne hydrocarboné(e) interrompu(e) par un ou plusieurs hétéroatomes, on entend un groupe ou chaîne comprenant un ou plusieurs hétéroatomes, chaque hétéroatome étant compris entre deux atomes de carbone dudit groupe ou de ladite, chaîne, ou entre un atome de carbone dudit groupe ou de ladite chaîne et un autre hétéroatome dudit groupe ou de ladite chaîne ou entre deux autres hétéroatomes dudit groupe ou de ladite chaîne.

Le ou les hétéroatomes peuvent être un atome d'azote, de soufre ou d'oxygène.

Selon un premier mode de réalisation, R₁ et R₂ représentent indépendamment H ou un groupe phényle.

Selon un deuxième mode de réalisation, R₂ représente un groupe phényle et R₁ représente H.

Le ou les composés de formule (I) sont avantageusement choisis parmi les composés dont le groupe A est choisi parmi
- le groupe 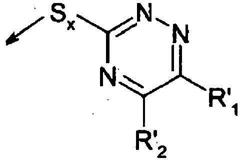
- le groupe 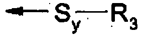
- le groupe 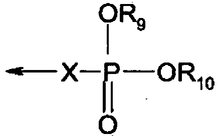
- le groupe 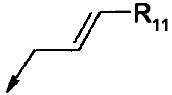
où x, R'₁, R'₂, y, R₃, X, R₉, R₁₀ et R₁₁ sont tels que définis précédemment.

Les groupes R₃ et R₆ à R₁₁ peuvent être choisis indépendamment parmi les groupes alkyles linéaires, ramifiés ou cycliques, les groupes aryles, les groupes aralkyles et les groupes alkylaryles, sous réserve d'éventuelles conditions explicitées précédemment pour chacun de ces groupements.

Les groupes R'₃, R₄ et R₅ peuvent être choisis indépendamment parmi les groupes alkyles linéaires, ramifiés ou cycliques, les groupes aryles, les groupes aralkyles et les groupes alkylaryles.

Les groupes X et Y peuvent être choisis indépendamment parmi les groupes alkylènes linéaires, ramifiés ou cycliques, les groupes arylènes et les groupes alkylarylènes.

Le cation Mⁿ⁺ peut être un cation métallique choisi parmi Zn²⁺, Cu²⁺, Na⁺, K⁺ et Li⁺.

Le cation Mⁿ⁺ peut être un cation non métallique tel qu'un ion ammonium, par exemple l'ion NMe₄⁺, NH₄⁺ ou H₃NMe⁺.

Selon un mode de réalisation particulier, R'₃, R₄ et R₅ représentent un groupe méthyle.

Selon un mode de réalisation particulier, R₆ représente un alkyle en C₁-C₄, plus préférentiellement un alkyle en C₁-C₃ et de manière très préférentielle, un groupe méthyle ou un groupe éthyle.

Selon un mode de réalisation particulier, R₇ représente un alkyle en C₁-C₄, plus préférentiellement un alkyle en C₁-C₃ et de manière très préférentielle, un groupe méthyle ou un groupe éthyle, ou bien R₇ représente un groupe aralkyle tel que par exemple un groupe benzyle.

Selon un mode de réalisation particulier, Y représente un alkylène en C₁-C₄, plus préférentiellement un alkylène en C₁-C₃ et de manière très préférentielle, un groupe propylène ou un groupe éthylène et Rg représente un alkyle en C₁-C₄, plus préférentiellement un alkyle en C₁-C₃ et de manière très préférentielle, un groupe méthyle ou un groupe éthyle.

Selon un mode de réalisation particulier, X représente un alkylène en C₁-C₄, plus préférentiellement un alkylène en C₁-C₃ et de manière très préférentielle, un groupe méthylène ou un groupe éthylène et R₉ et R₁₀ représentent indépendamment un alkyle en C₁-C₄, plus préférentiellement un alkyle en C₁-C₃ et de manière très préférentielle, un groupe méthyle ou un groupe éthyle.

Selon un mode de réalisation particulier, R₁₁ représente H.

A titre de composés de formule (I) particuliers, on peut citer les composés suivants :

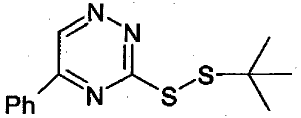

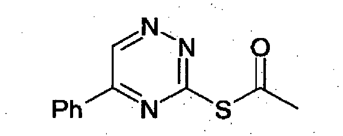

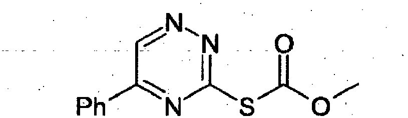

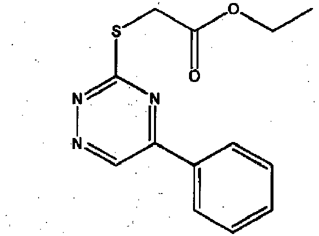

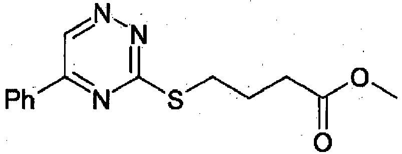

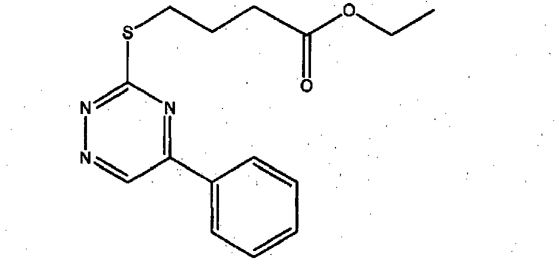

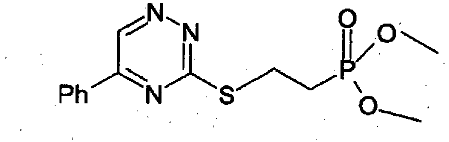

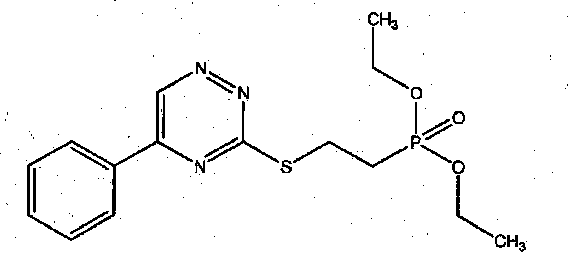

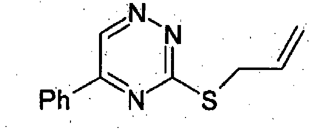

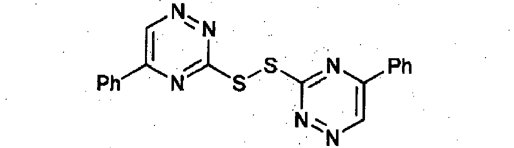

Le ou les composés de formule (I) et/ou (II) représentent généralement de 0,1 à 7 pce, de préférence de 0,2 à 7 pce, de préférence encore de 0,5 à 7 pce, mieux de 0,5 à 5 pce.

Le système de vulcanisation de la composition selon l'invention peut également comprendre un ou plusieurs accélérateurs primaires additionnels, en particulier les composés de la famille des thiurames, les dérivés dithiocarbamates de zinc, les sulfénamides, les guanidines ou les thiophosphates.

### II-4. Additifs divers

La composition de caoutchouc selon l'invention peut comporter également tout ou partie des additifs usuels habituellement utilisés dans les compositions d'élastomères destinées à la fabrication de pneumatiques, en particulier de bandes de roulement, comme par exemple des plastifiants ou des huiles d'extension, que ces derniers soient de nature aromatique ou non-aromatique, des pigments, des agents de protection tels que cires anti-ozone (telle que la Cire Ozone C32 ST), anti-ozonants chimiques, anti-oxydants (tel que la 6-paraphénylènediamine), des agents anti-fatigue, des résines renforçantes, des accepteurs (par exemple résine phénolique novolaque) ou des donneurs de méthylène (par exemple HMT ou H3M) tels que décrits par exemple dans la demande WO 02/10269.

De préférence, la composition selon l'invention comporte, à titre d'agent plastifiant préférentiel non aromatique ou très faiblement aromatique, au moins un composé choisi dans le groupe constitué par les huiles naphténiques, paraffiniques, huiles MES, huiles TDAE, les esters (en particulier trioléates) de glycérol, les résines plastifiantes hydrocarbonées présentant une haute Tg de préférence supérieure à 30°C, et les mélanges de tels composés.

La composition selon l'invention peut également contenir, en complément des agents de couplage, dés activateurs de couplage de la charge inorganique renforçante ou plus généralement des agents d'aide à la mise en oeuvre susceptibles de manière connue, grâce à une amélioration de la dispersion de la charge inorganique dans la matrice de caoutchouc et à un abaissement de la viscosité des compositions, d'améliorer leur faculté de mise en oeuvre à l'état cru, ces agents étant par exemple des silanes hydrolysables tels que des alkylalkoxysilanes (notamment des alkyltriéthoxysilanes), des polyols, des polyéthers (par exemple des polyéthylèneglycols), des amines primaires, secondaires ou tertiaires (par exemple des trialcanol-amines), des POS hydroxylés ou hydrolysables, par exemple des α,ω-dihydroxy-polyorganosiloxanes (notamment des α,ω-dihydroxy-polydiméthylsiloxanes), des acides gras comme par exemple l'acide stéarique.

### II-5. Fabrication des compositions de caoutchouc

La composition de caoutchouc selon l'invention est fabriquée dans des mélangeurs appropriés, en utilisant généralement deux phases de préparation successives selon une procédure générale bien connue de l'homme du métier : une première phase de travail ou malaxage thermo-mécanique (parfois qualifiée de phase "non-productive") à haute température, jusqu'à une température maximale comprise entre 110°C et 190°C, de préférence entre 115°C et 150°C et plus préférentiellement encore entre 115°C et 140°C, suivie d'une seconde phase de travail mécanique (parfois qualifiée de phase "productive") à plus basse température, typiquement inférieure à 110°C, par exemple entre 40°C et 100°C, phase de finition au cours de laquelle peut être incorporé le système de réticulation ou vulcanisation.

Selon un mode de réalisation préférentiel de l'invention, tous les constituants de base de la composition de l'invention, à l'exception du système de vulcanisation, à savoir la ou les charges renforçantes, l'agent de couplage le cas échéant, sont incorporés de manière intime, par malaxage, à l'élastomère diénique ou aux élastomères diéniques au cours de la première phase dite non-productive, c'est-à-dire que l'on introduit dans le mélangeur et que l'on malaxe thermomécaniquement, en une ou plusieurs étapes, au moins ces différents constituants de base jusqu'à atteindre la température maximale comprise entre 110°C et 190°C, de préférence comprise entre 115°C et 150°C.

Les deux phases peuvent être réalisées de manière consécutive sur un même mélangeur ou être séparées par une étape de refroidissement à une température inférieure à 100°C, la dernière étape étant alors réalisée sur un deuxième mélangeur.

A titre d'exemple, la première phase (non-productive) est conduite en une seule étape thermomécanique au cours de laquelle on introduit, dans un mélangeur approprié tel qu'un mélangeur interne usuel, tous les constituants nécessaires, les éventuels agents de mise en oeuvre complémentaires et autres additifs divers, à l'exception du système de vulcanisation. La durée totale du malaxage, dans cette phase non-productive, est de préférence comprise entre 1 et 15 min. Après refroidissement du mélange ainsi obtenu au cours de la première phase non-productive, on incorpore alors le système de vulcanisation à basse température, généralement dans un mélangeur externe tel qu'un mélangeur à cylindres; le tout est alors mélangé (phase productive) pendant quelques minutes, par exemple entre 2 et 15 min.

La composition finale ainsi obtenue est ensuite calandrée par exemple sous la forme d'une feuille ou d'une plaque, notamment pour une caractérisation au laboratoire, ou encore extrudée sous la forme d'un profilé de caoutchouc utilisable par exemple comme une bande de roulement de pneumatique pour véhicule tourisme.

### II-6. Composés 1,2,4-triazines particuliers

L'invention a encore pour objet un composé 1,2,4-triazine de formule 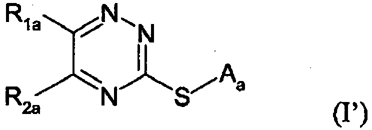 où
R₁ₐ et R₂ₐ représentent indépendamment H, un halogène, un groupe choisi par les groupes alcoxy, et (C₁-C₁₀)alkylthio, ou un groupe hydrocarboné en C₁-C₂₅ choisi parmi les groupes alkyles linéaires, ramifiés ou cycliques, les groupes aralkyles, les groupes alkylaryles et les groupes aryles, et éventuellement interrompus par un ou plusieurs hétéroatomes, R₁ₐ pouvant former un cycle avec R₂ₐ,
- Aₐ est choisi parmi
   - un groupe 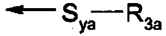 où R₃ₐ représente un groupe hydrocarboné en C₁-C₁₈, éventuellement interrompu par un ou plusieurs hétéroatomes, à la condition que R₃ₐ ne comporte pas de 1,2,4-triazine, et à la condition que le composé (I') ne soit pas de formule 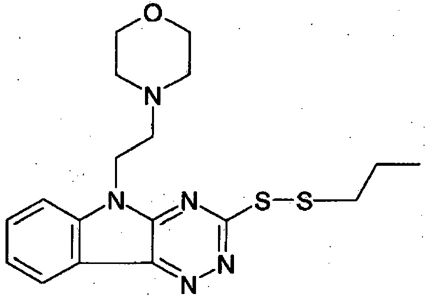 yₐ est un entier supérieur ou égal à 1, préférentiellement inférieur ou égal à 10, plus préférentiellement inférieur ou égal à 8 et très préférentiellement inférieur ou égal à 6,
   - un groupe 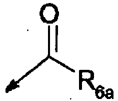 où R₆ₐ est un groupe hydrocarboné en C₁-C₁₈, éventuellement interrompu par un ou plusieurs hétéroatomes, étant entendu que R₆ₐ est différent d'un groupe phényle éventuellement substitué, et étant entendu que si R₆ₐ représente un groupe méthyle, R₁ₐ et R₂ₐ ne forment pas de cycle et R₁ₐ et R₂ₐ ne représentent pas respectivement un méthyle et un hydroxyle,
   - un groupe 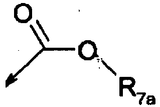 où R₇ₐ est un groupe hydrocarboné en C₁-C₁₈, éventuellement interrompu par un ou plusieurs hétéroatomes, étant entendu que si R₇ₐ représente un groupe éthyle, R₁ₐ et R₂ₐ ne forment pas de cycle,
   - un groupe 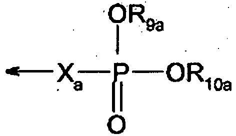 où Xₐ est une chaîne hydrocarbonée en C₁-C₁₈, éventuellement interrompue ou substituée par un ou plusieurs hétéroatomes ;
R₉ₐ et R₁₀ₐ représentent indépendamment un groupe hydrocarboné en C₁-C₁₈, éventuellement interrompu par un ou plusieurs hétéroatomes.

Par groupe alkyle cyclique, on entend un groupe alkyle constitué d'un ou plusieurs cycles.

Par groupe ou chaîne hydrocarboné(e) interrompu(e) par un ou plusieurs hétéroatomes, on entend un groupe ou chaîne comprenant un ou plusieurs hétéroatomes, chaque hétéroatome étant compris entre deux atomes de carbone dudit groupe ou de ladite chaîne, ou entre un atome de carbone dudit groupe ou de ladite chaîne et un autre hétéroatome dudit groupe ou de ladite chaîne ou entre deux autres hétéroatomes dudit groupe ou de ladite chaîne.

Le ou les hétéroatomes peuvent être un atome d'azote, de soufre ou d'oxygène.

Selon un premier mode de réalisation, R₁ₐ et R₂ₐ représentent indépendamment H ou un groupe phényle.

Selon un deuxième mode de réalisation, R₂₈ représente un groupe phényle et R₁ₐ représente H.

Avantageusement, le composé 1,2,4-triazine de formule (I') est tel que Aa est choisi parmi :
- le groupe 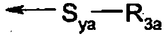 et
- le groupe 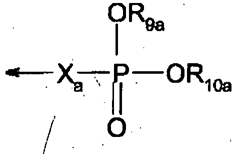
où yₐ, R₃ₐ, Xₐ, R₉ₐ et R₁₀ₐ sont tels que définis précédemment.

Les groupes R₃ₐ, R₆ₐ, R₇ₐ et R₉ₐ à R₁₀ₐ peuvent être choisis indépendamment parmi les groupes alkyles linéaires, ramifiés ou cycliques, les groupes aryles, les groupes aralkyles et les groupes alkylaryles, sous réserve d'éventuelles conditions explicitées précédemment pour chacun de ces groupements.

De préférence, le groupe R'₃ₐ représente un groupe CR'₃ₐR₄ₐR₅ₐ, où R'₃ₐ, R₄ₐ et R₅ₐ représentent indépendamment H ou un groupe en C₁-C₁₇ éventuellement interrompu par un ou plusieurs hétéroatomes,

Les groupes R'₃ₐ, R₄ₐ et R₅ₐ peuvent être choisis indépendamment parmi les groupes alkyles linéaires, ramifiés ou cycliques, les groupes aryles, les groupes aralkyles et les groupes alkylaryles.

Le groupe Xₐ peut être choisi parmi les groupes alkylènes linéaires, ramifiés ou cycliques, les groupes arylènes et les groupes alkylarylénes.

Selon un mode de réalisation particulier, R'₃ₐ, R₄ₐ et R₅ₐ représentent un groupe méthyle.

Selon un mode de réalisation particulier, R₇ₐ représente un alkyle en C₁-C₄, plus préférentiellement un alkyle en C₁-C₃ et de manière très préférentielle, un groupe méthyle ou un groupe éthyle, ou bien R₇ₐ représente un groupe aralkyle tel que par exemple un groupe benzyle.

Selon un mode de réalisation particulier, Xa représente un alkylène en C₁-C₄, plus préférentiellement un alkylène en C₁-C₃ et de manière très préférentielle, un groupe méthylène ou un groupe éthylène et R₉ₐ et R₁₀ₐ représentent indépendamment un alkyle en C₁-C₄, plus préférentiellement un alkyle en C₁-C₃ et de manière très préférentielle, un groupe méthyle ou un groupe éthyle.

### II-7. Préparation des accélérateurs de formule générale (I), (II) et (I')

L'homme du métier sait préparer les composés 1,2,4-triazines de formule générale (I), lorsque le groupement A représente un atome d'hydrogène, auquel cas les 1,2,4-triazines sont substituées par un thiol. Ce thiol peut être salifié par des techniques connues de l'homme du métier pour obtenir les composés thiolates, de formule générale (II).

A partir des thiols ou thiolates décrits ci-dessus et selon la nature du groupement A, l'homme du métier peut obtenir les composés de formule générale (I) ou (I') comme indiqué ci-dessous :
- Lorsque le radical A représente un groupe 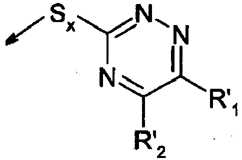 tel que défini précédemment, le composé peut être préparé par un couplage oxydant.
   A une solution du thiol précurseur, on ajoute un agent oxydant organique ou inorganique (par exemple le diisopropylazodicarboxylate ou le persulfate d'ammonium, ou encore de l'iode ou une solution d'hypochlorite de sodium). Par exemple, ce type de mode opératoire est décrit dans les documents suivants :
   - avec NaOCl par Mathes dans US2196607 (1940),
   - avec (NH₄)₂S₂O₈ dans Stewart, Mathes, J. of Org. Chem., 1949, vol.14, p. 1111 - 1117.

   Par cette réaction on obtient les dérivés dans lesquels x = 1. Pour obtenir les composés dans lesquels x est supérieur à 1, on peut insérer des atomes de soufre dans la liaison soufre - soufre existante, par exemple par réaction avec le chlorure de triphénylthiosulfényl dans le dichlorométhane en présence d'acide acétique, comme proposé par exemple dans Tetrahedron Letters, Volume 41, Issue 37, September 2000, Pages 7169-7172*.*
- Lorsque le radical A ou Aa représente un groupe 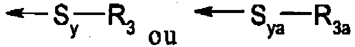 tel que défini précédemment, la voie de synthèse peut être similaire à celle décrite pour les composés précédents.
   Par exemple Birch décrit le couplage oxydant du méthylmercaptan avec le tertbutyl mercaptan en présence hexacyanoferrate III de potassium (Birch et al. Journal of the Institute of Petroleum, 1953, vol. 39, p. 206,210*).*
   On peut aussi suivre une autre procédure consistant à réaliser une réaction de redistribution d'un disulfure symétrique en présence d'un alkylmercaptan tel que décrit par exemple par Brzezinska, Ewa; Ternay, Andrew L., Jr. dans l'article Disulfides Syntheses Using 2,2'-Dithiobis(benzothiazole), Journal of Organic Chemistry (1994), 59, 8239-8244*.* Cette méthode consiste à faire une réaction dans le chloroforme entre un disulfure symétrique et un mercaptan comme par exemple le tertbutylmercaptan ce qui conduit à une redistribution.
   Par cette réaction on obtient les dérivés dans lesquels y = 1. Pour obtenir les composés dans lesquels y est supérieur à 1, on peut insérer des atomes de soufre dans la liaison soufre - soufre existante, par exemple par réaction avec le chlorure de triphénylthiosulfényl dans le dichlorométhane en présence d'acide acétique, comme proposé par exemple dans Tetrahedron Letters, Volume 41, Issue 37, September 2000, Pages 7169-7172*.*
- Lorsque le radical A ou Aa représente un groupe 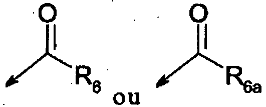 tel que défini précédemment, ce composé peut être préparé selon une réaction de thioesterification entre le thiol ou son thiolate correspondant avec un acide carboxylique ou ses dérivés (chlorure d'acide, anhydride) portant le radical R₆.
   Par exemple, Jan Larsen et Christine Lenoir dans Organic Syntheses, Coll. Vol. 9, p.72 (1998*),* décrivent une réaction de thioesterification entre un thiol et un chlorure d'acide en présence de triéthylamine pour piéger l'acide chlorhydrique formé, la réaction étant menée dans le dichlorométhane entre 0 et 10°C.
- Lorsque le radical A ou Aa représente un groupe 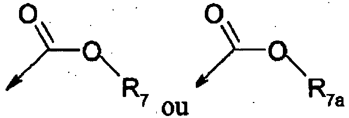 tel que défini précédemment, ce composé peut être préparé par réaction d'addition élimination entre le thiol et un chloroformate d'alkyle portant le radical R₇ ou R₇ₐ qui peut être lui-même, soit disponible commercialement, soit préparé par réaction entre un alcool et le phosgène ou un de ses dérivés. Une base organique ou inorganique peut être ajoutée pour piéger l'acide chlorhydrique formé.
   Cette approche est décrite par Taylor, Roger dans le Journal of the Chemical Society, Perkin Transactions 2: Physical Organic Chemistry (1972-1999), 1983, p. 291 - 296*.* Les auteurs font réagir l'éthylmercaptan avec le chloroformiate de méthyle en présence de pyridine.
   Au lieu d'utiliser le thiol, il est possible d'utiliser le thiolate correspondant. Cette approche est décrite par Suzuki, Shigenori; Hisamichi, Kanehiko; Endo, Katsuya dans Heterocycles, 1993, vol. 35, # 2 p. 895 - 900*.* Les auteurs font réagir le methylthiolate de sodium avec du chloroformiate de méthyle pour obtenir le dérivé thiocarbonate.
   Une autre voie de synthèse consiste à faire réagir le thiol ou son thiolate avec le phosgène (Chen, H. W. et al. Journal of the American Chemical Society, 1978, vol. 100, p. 2370 - 2375*)* ou un de ses dérivés tel que le triphosgène (WO2008/038175) éventuellement en présence d'une base inorganique ou organique telle qu'une amine tertiaire par exemple pour former l'intermédiaire chlorothioformate. Cet intermédiaire peut ensuite réagir avec un alcool en présence d'une base organique telle qu'une amine tertiaire pour former le composé thiocarbonate. Cette approche est par exemple décrite dans Organic Syntheses, Coll. Vol. 5, p.166 (1973*);* Vol. 44, p.20 (1964*).*
- Lorsque le radical A représente un groupe 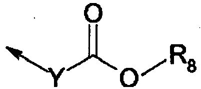 tel que défini précédemment, ce composé peut être préparé par substitution nucléophile entre le thiol ou son thiolate et un ester substitué par un groupement halogéné et portant les radicaux Y et R₈ comme par exemple le bromoéthylacetate ou le 4 bromo méthylbutyrate.
   Par exemple, la réaction entre la 3-mercapto-5,6-diphényl-1,2,4-triazine et l'éthyle 3-bromo-propionate dans l'acétate d'éthyle en présence de triéthylamine pour piéger l'acide bromhydrique formé est décrite par Bhalla, M.; Srivastava, V.K.; Bhalla, T.N.; Shanker, K. dans Bollettino Chimico Farmaceutico (1995), 134(1), 9-15*.*
- Lorsque le radical A ou Aa représente un groupe 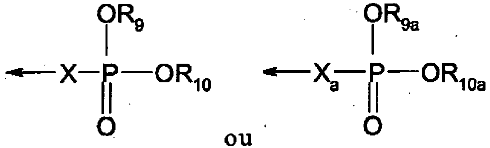 tel que défini précédemment, ce composé peut être préparé par substitution nucléophile entre le thiol ou son thiolate et un dérivé halogéné d'un phosphonate ou d'un acide phosphonique portant les radicaux X, R₉ et R₁₀ ou Xa, R₉ₐ et R₁₀ₐ, et plus particulièrement avec un bromo ou chloro alkyl phosphonate.
   Cette réaction est décrite par exemple pour la réaction entre le diethyl (2-bromo-éthyl)phosphonate et l'éthanethiolate de sodium dans un éther (Mikolajczyk, Marian; Costisella, Burkhard; Grzejszczak, Slawomir, Tetrahedron, 1983, vol. 39, # 7 p. 1189 - 1193)*.*
- Lorsque le radical A représente un groupe 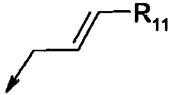 tel que défini précédemment, ce composé peut être préparé par substitution nucléophile entre le thiol ou son thiolate et un halogénure d'allyle portant le radical R₁₁ et par exemple entre le thiol et le bromure d'allyle.
   Par exemple la réaction entre une triazine thiol et un halogénure d'allyle en présence de potasse dans l'alcool est décrite dans le brevet BE 503980 de la Société Belge de l'azote et des produits chimiques du Marly (1951).

Un exemple de composé de formule (I) est la 1,2,4-Triazine-3-thione 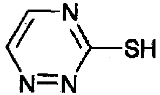 dont la synthèse est décrite par exemple dans le brevet BE503980.

Un autre exemple de composé de formule (I) la 3-mercapto-5,6-diphényl-1,2,4-triazine de formule 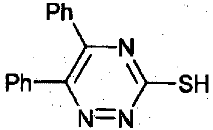 décrite dans le document Some mercaptotriazines, Gianfurco, Maurizio; Romeo, Aurelio, Gazzetta Chimica Italiana (1953), 82, 429-34.

On peut encore citer le composé de formule 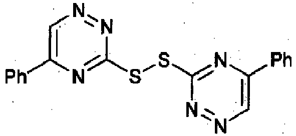 décrit dans le document Syntheses and structure of some 5-substituted 2,3-dihydro-1,2,4-triazine-3-thiones, Tisler, M, Croatica Chemica Acta (1960), 32, 123-32

On peut encore citer le composé de formule 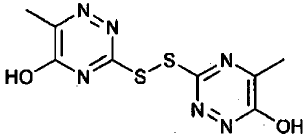 décrit dans le document *Oxidation of substituted thioxo-1,2,4-triazinones,* Mironovich, L. M.; Salistyi, S. M., Ukrainskii Khimicheskii Zhurnal (Russian Edition) (1996), 62(1-2), 131-133*.*

On peut encore citer le composé de formule 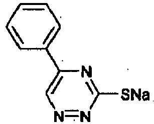 décrit dans le document *Antifungal properties of a novel 1,2,4-triazine derivative I 319,* Wieczorek, Jadwiga; Mordarski, Marian; Rykowski, Andrzej; Nantka-Namirski, Pawel, Archivum Immunologiae et Therapiae Experimentalis (1980), 28(5), 727-33 On peut encore citer le composé de formule 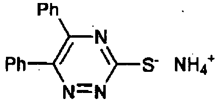 décrit dans le document *Synthesis and neuropharmacological activity of 1,2,4-triazine-3-thione derivatives,* Smagin, S. S.; Bogachev, V. E.; Yakubovskii, A. K.; Metkalova, S. E.; Privol'neva, T. P.; Chugunov, V. V.; Lavretskaya, E. F., Khimiko-Farmatsevticheskii Zhurnal (1975), 9(4), 11-15*.*

On peut encore citer les composés de formule 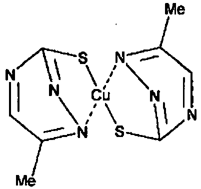 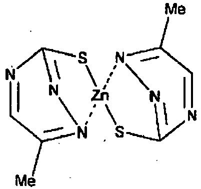 décrits dans le document *Use of asymmetric triazines in analytical chemistry. III*. *Constitution of asymmetric monoalkyl*-*3-mercaptotriazine with some metals,* Kralovsky, Josef; Jenik, Josef; Renger, Frantisek, Sbornik Vedeckych Praci - Vysoka Skola Chemickotechnologicka Pardubice (1969), No. 19(Pt. 1), 79-84*.*

### III. Exemples de réalisation de l'invention

### III-1 Exemples de synthèses de composés

### 1. Synthèse du composé A 3-(allylthio)-5-phényl-1,2,4-triazine

La préparation du 3-(allylthio)-5-phényl-1,2,4-triazine (composé A) est effectuée à partir du 5-phényl-1,2,4-triazine-3-thiol et du bromure d'allyle, selon le schéma de synthèse suivant :

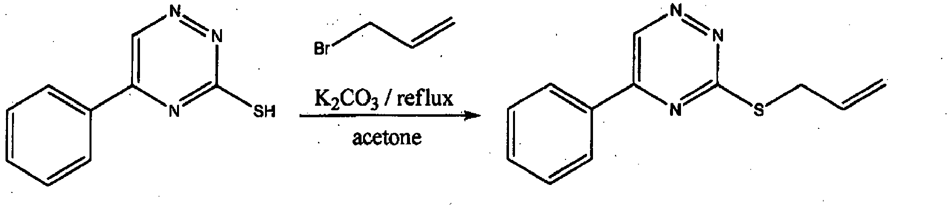

Le 3-(allylthio)-5-phényl-1,2,4-triazine est décrit dans la littérature: Rudakov, B. V.; Kim, D. G.; Alekseev, S. G.; Charushin, V. N.; Shorshnev, S. V.; Russian Journal of Organic Chemistry; vol. 33; nb. 7; (1997); p. 1033 - 1036; Zhurnal Organicheskoi Khimii; vol. 33; nb. 7; (1997); p. 1103-1106).

A une solution de 5-phényl-1,2,4-triazine-3-thiol (3,80 g, 0,020 mol, 90% mol.) dans 25 mL d'acétone est ajouté, en une fois, du K₂CO₃ (1,61 g, 0,011 mol, Aldrich, >99%). Le milieu réactionnel est agité pendant 1,5 heure à température ambiante (22-24 °C). Une solution du bromure d'allyle (2,56 g, 0,021 mol, Acros, 99%) dans 15 mL d'acétone est ensuite ajoutée pendant 10 minutes. Le milieu réactionnel est agité pendant 40 minutes à reflux puis refroidi lentement (40-50 minutes) jusqu'à température ambiante. Le précipité est filtré, le filtrat est concentré sous pression réduite (8-10 mbar, 23 °C).

Une huile brune (4,04 g, 0,017 mol, rendement 88 %) est obtenue.

La pureté molaire est supérieure à 94 % (RMN ¹H).

CCM : R_{f} = 0,6 (SiO₂; heptane : EtOAc = 2 : 3; révélation par UV et I₂).

Les déplacements chimiques obtenus par RMN ¹H et ¹³C dans le DMSO-d6 sont donnés dans le tableau ci-dessous. La calibration est réalisée sur le DMSO (2,44ppm en ¹H et à 39,5 ppm en ¹³C).

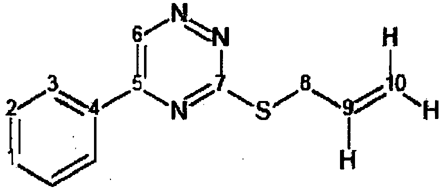

| N° atomes | δ du ¹H(ppm) | δ du ¹³C(ppm) |
|---|---|---|
| 1 | 7,61 | 132,6 |
| 2 | 7,55 | 129,3 |
| 3 | 8,25 | 127,8 |
| 4 | / | 133,2 |
| 5 | / | 154,1 |
| 6 | 9,77 | 142,9 |
| 7 | / | 171,5 |
| 8 | 3,92 | 32,4 |
| 9 | 5,96 | 132,8 |
| 10 | 5,10 + 5,33 | 118,3 |

Caractérisation en spectroscopie de masse ID/IC (Injection Directe / ionisation chimique) :
Environ 20 mg d'échantillon a été mis en solution dans 2 ml d'acétone pour l'analyse par CPG/SM/IE et ID/IC.
m/z : 258 ([M+C²H⁵]⁺), 230 ([M+H]⁺)

### 2. Synthèse du composé B : 1,2-Bis(5-phényl-1,2,4-triazin-3-yl)disulfure (ou 5-phényl-3-[(5-phenyl-1,2,4-triazin-3-yl)dithio]-1,2,4-triazine)

La préparation de ce composé est effectuée à partir du 5-phényl-1,2,4-triazine-3-thiol selon le schéma de synthèse suivant qui repose sur un couplage oxydant :

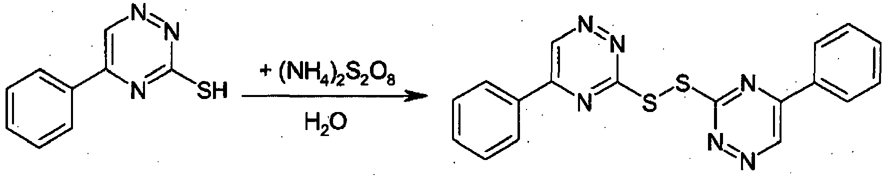

Le 5-phényl-1,2,4-triazine-3-thiol est commercial (numéro CAS [15969-28-5]) et peut être obtenu selon des procédures décrites dans les documents suivants :
1. Daunis, J. et al.; Bulletin de la Société Chimique de France; 1969, 10; 3675-3678.
2. Joshi, K. C.; Dubey, K. Dandia, A. Heterocycles, (1981), 16(9); 1545 - 1553.

Au mélange de 5-phényl-1,2,4-triazine-3-thiol (3,21 g, 0,017 mol) et d'eau (100 mL) est ajouté en une fois le persulfate d'ammonium (5,80 g, 0,025 mol.). Le milieu réactionnel est agité pendant 15 minutes au reflux. Après retour à température ambiante le précipité est filtré et lavé 3 fois par de l'eau (150 mL). Après séchage à température ambiante, un solide jaune (2,78 g, 0,007 mol., rendement 87 %) de point de fusion 185 °C est obtenu. La pureté molaire estimée par RMN ¹H est de 95 %.

La caractérisation complète du produit est réalisée par RMN. Les déplacements chimiques obtenus par RMN ¹H et ¹³C dans le DMSO-d6 sont donnés dans le tableau ci-dessous. La calibration est réalisée sur le DMSO (2,44 ppm en ¹H et à 39,5 ppm en ¹³C).

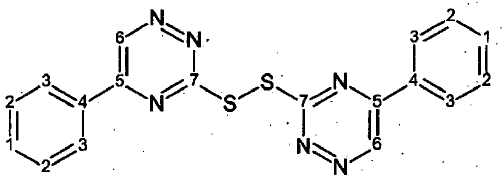

Les résultats sont donnés dans le tableau ci-dessous.

| Atome | δ ¹H (ppm) + mult. | δ ¹³C (ppm) |
|---|---|---|
| 1 | 7,58 (t) | 133,3 |
| 2 | 7,48 (t) | 129,4 |
| 3 | 8,14(d) | 127,9 |
| 4 | - | 132,2 |
| 5 | - | 155,2 |
| 6 | 9,93 (s) | 144,7 |
| 7 | - | 168,9 |

### 3. Synthèse du composé C : 3-(tert-butyldisulfanyl)-5-phényl-1,2,4-triazine

La préparation du 3-(tert-butyldisulfanyl)-5-phényl-1,2,4-triazine (composé C) est effectuée en deux étapes à partir du 5-phényl-1,2,4-triazine-3-thiol :
Etape 1 : 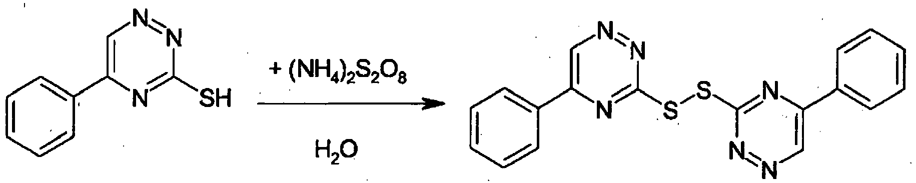
   L'obtention du 1,2-bis(5-phényl-1,2,4-triazin-3-yl)disulfure (composé B) est décrite précédemment.
Etape 2 :

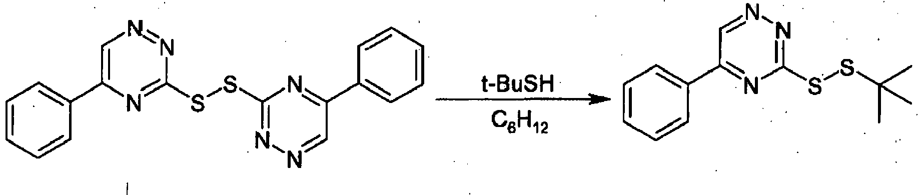

### Mode opératoire de la 2^{ème} étape :

À un mélange de 1,2-bis(5-phényl-1,2,4-triazin-3-yl)disulfure (18,5 g, 0,049 mol) et de tert-butanethiol (4,88 g, 0,054 mol) dans le cyclohexane (600 mL) à 50°C est ajoutée une solution de triéthylamine (5,47 g, 0,051 mol) dans 50 mL de cyclohexane. Le milieu réactionnel est maintenu à 50-53°C pendant 13 heures. Le milieu réactionnel est filtré à 45°C sur Célite® puis lavé par l'éther de pétrole (deux fois par 50 mL). Le filtrat est condensé sous pression réduite (T_{bain} 40 °C, 8-15 mbar) et un solide rouge (11,82 g, 0,043 mol, rendement 87 %) de point de fusion 99°C est obtenu.

La pureté molaire est supérieure à 94 % (RMN ¹H).

### Caractérisation RMN :

Les déplacements chimiques obtenus par RMN ¹H et ¹³C dans le DMSO-d6 sont donnés dans le tableau ci-dessous. La calibration est réalisée sur le DMSO (2,44ppm en ¹H et à 39,5 ppm en ¹³C).

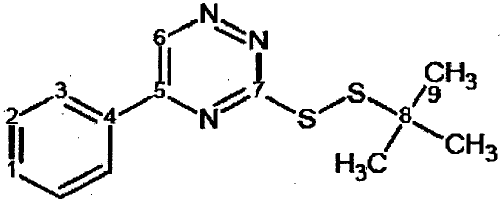

| N° | δ ¹H (ppm) | δ ¹³C (ppm) |
|---|---|---|
| 1 | 7,63 | 134,0 |
| 2 | 7,58 | 129,3 |
| 3 | 8,32 | 127,7 |
| 4 | / | 132,3 |
| 5 | / | 154,5 |
| 6 | 9,89 | 144,1 |
| 7 | / | 170,9 |
| 8 | / | 49,1 |
| 9 | 1,30 | 29,2 |

### 4. Synthèse du composé D : diéthyl 2-(5-phényl-1,2,4-triazin-3-ylthio)éthylphosphonate

Le diéthyl 2-(5-phényl-1,2,4-triazin-3-ylthio)éthylphosphonate est préparé en une étape à partir du 5-phényl-1,2,4-triazine-3-thiol selon le schéma suivant :

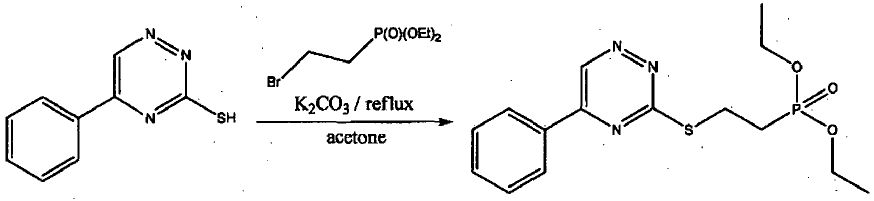

A une solution de 5-phényl-1,2,4-triazine-3-thiol (5,40 g, 0,029 mol, , ∼ 90 % mol.) dans 50 mL d'acétone est ajouté, en une fois, du K₂CO₃ (3,88 g, 0,028 mol, Aldrich, >99%). Le milieu réactionnel est agité pendant 20 minutes à température ambiante (22-24 °C) puis du diéthyle de 2-bromoéthylphosphonate (6,90 g, 0,028 mol, Aldrich, 97%) est ajouté goutte à goutte sur une durée de 3 minutes. Le milieu réactionnel est agité pendant une heure au reflux et est ensuite refroidi lentement (2 heures) jusqu'à température ambiante.

Le milieu réactionnel est filtré (élimination des sels résiduels) et les sels solides sont lavés sur le filtre par 20 mL d'acétone. Le filtrat est concentré sous pression réduite (10-13 mbar, 23°C) et le solide résiduel est séché sous pression réduite jusqu'à obtention d'une masse constante

Un solide jaune (8,94 g; 0,025 mol; rendement 90%) de point de fusion 78-79 °C est obtenu.

La pureté molaire est supérieure à 91 % (RMN ¹H).

CCM : R_{f} = 0,30 (SiO₂; EtOAc; révélation par UV et I₂).

### Caractérisation RMN :

Les déplacements chimiques obtenus par RMN ¹H et ¹³C dans le DMSO-d6 sont donnés dans le tableau ci-dessous. La calibration est réalisée sur le DMSO (2,44ppm en ¹H, à 39,5 ppm en ¹³C et la calibration ³¹P avec sr=0 ; sr : spectrum reference).

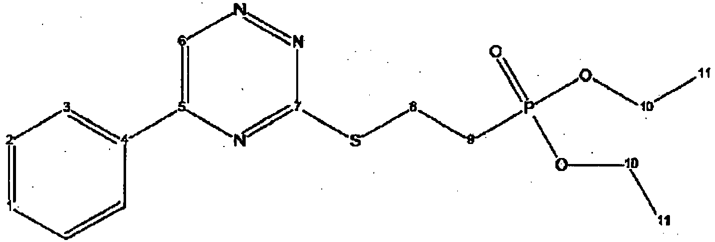

| N° | δ ¹H (ppm) | δ ¹³C (ppm) |
|---|---|---|
| 1 | 7,62 | 132,9 |
| 2 | 7,55 | 129,1 |
| 3 | 8,28 | 127,9 |
| 4 | - | 132,8 |
| 5 | - | 154,1 |
| 6 | 9,78 | 142,8 |
| 7 | - | 171,3 |
| 8 | 3,35 | ∼23,3 |
| 9 | 2,24 | ∼24,8 |
| 10 | 3,99 | 61,1 |
| 11 | 1,20 | 16,2 |

Le déplacement chimique du ³¹P est 27,9 ppm.

Caractérisation en spectroscopie de masse ID/IC (Injection Directe / ionisation chimique) :
Environ 20 mg d'échantillon a été mis en solution dans 2 ml d'acétone pour l'analyse par CPG/SM/IE et ID/IC.
m/z : 354 ([M+H]⁺)

### 5. Synthèse du composé E : éthyl 2-(5-phényl-1,2,4-triazin-3-ylthio)acétate

Le composé E est préparé selon le schéma de synthèse suivant :

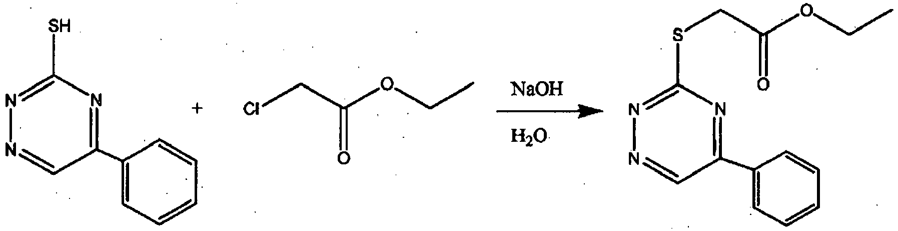

À une solution de 5-phényl-1,2,4-triazine-3-thiol (14,84 g, 0,078 mol) et d'hydroxyde de sodium (3,136 g, 0,078 mol) dans 450 mL d'eau est ajouté du chloroacétate d'éthyle (9,608 g, 0,078 mol) goutte à goutte à température ambiante. Le milieu réactionnel est ensuite agité intensivement pendant 2 heures, puis filtré et lavé par l'eau (2L).

Après cristallisation dans 300 mL d'isopropanol (soluble à chaud à environ + 50 °C), un solide jaune pâle (14,22 g, 0,052 mol, rendement 67 %) de point de fusion 104 °C est obtenu.

La pureté molaire est supérieure à 98 % (RMN ¹H).

### Caractérisation RMN :

Les déplacements chimiques obtenus par RMN ¹H et ¹³C dans l'acétone-d6 sont donnés dans le tableau ci-dessous. La calibration est réalisée sur l'acétone (1,98 ppm en ¹H et à 29,8 ppm en ¹³C).

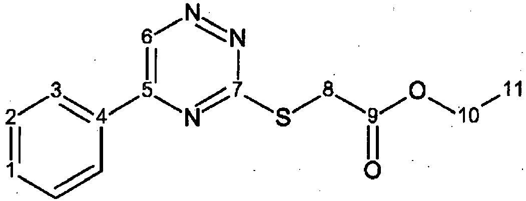

| N° | δ ¹H (ppm) | δ ¹³C (ppm) |
|---|---|---|
| 1 | 7,61 | 133,3 |
| 2 | 7,56 | 129,8 |
| 3 | 8,27 | 128,4 |
| 4 | - | 133,8 |
| 5 | - | 155,1 |
| 6 | 9,65 | 143,3 |
| 7 | - | 172,4 |
| 8 | 4,10 | 33,1 |
| 9 | - | 168,9 |
| 10 | 4,11 | 61,8 |
| 11 | 1,16 | 14,2 |

### 6. Synthèse du composé F : éthyl 4-(5-phényl-1,2,4-triazin-3-ylthio)butanoate

La préparation du composé F s'effectue selon le schéma réactionnel suivant :

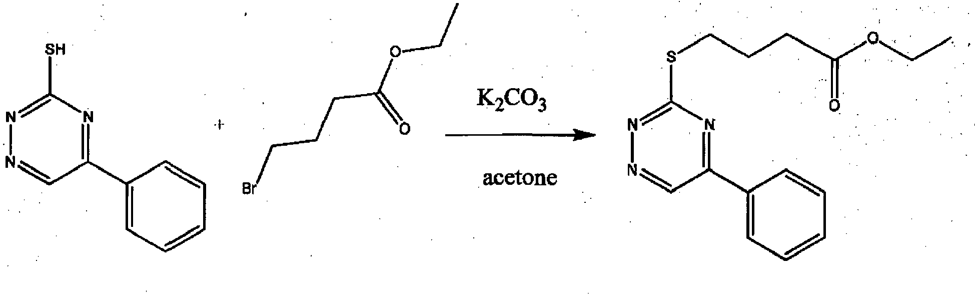

À une solution de 5-phényl-1,2,4-triazine-3-thiol (8,412 g, 0,044 mol) dans l'acétone (250 mL) est ajouté du K₂CO₃ (6,14 g, 0,044 mol). Le milieu réactionnel est ensuite agité 15 minutes et de l'éthyle de 4-bromobutyrate 95 % (9,13 g, 0,044 mol) est ajouté goutte à goutte. Le milieu réactionnel est porté au reflux pendant 2,5 heures, puis le mélange est refroidi jusqu'à 20 °C et est filtré. L'évaporation du solvant sous pression réduite fournit une huile noire.

Après cristallisation dans 200 mL d'éthanol, un solide jaune pâle (8,744 g, 0,029 mol, rendement 65%) de point de fusion 51-52 °C est obtenu.

La pureté molaire est supérieure à 98 % (RMN ¹H).

CCM : R_{f} = 0,66 (SiO₂; cyclohexane EtOAc = 1 : 1; révélation par UV et I₂).

### Caractérisation RMN :

Les déplacements chimiques obtenus par RMN ¹H et ¹³C dans le chloroforme-d1 sont donnés dans le tableau ci-dessous. La calibration est réalisée sur le chloroforme (7,2 ppm en ¹H et à 77 ppm en ¹³C).

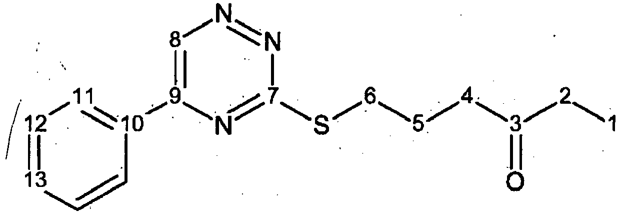

| N° | δ ¹H (ppm) | δ ¹³C (ppm) |
|---|---|---|
| 1 | 1,19 | 14,2 |
| 2 | 4,08 | 60,4 |
| 3 | / | 172,6 |
| 4 | 2,47 | 33,1 |
| 5 | 2,12 | 24,4 |
| 6 | 3,32 | 29,9 |
| 7 | / | 173,0 |
| 8 | 9,31 | 141,9 |
| 9 | / | 154,6 |
| 10 | / | 133,0 |
| 11 | 8,09 | 127,6 |
| 12/13 | 7,45 - 7,58 | 129,3 + 132,6 |

### III-2. Préparation des compositions

On procède pour les essais qui suivent de la manière suivante: on introduit dans un mélangeur interne, rempli à 70% et dont la température initiale de cuve est d'environ 90°C, le ou les élastomères diéniques, la ou les charges renforçantes, l'agent de couplage éventuel puis, après une à deux minutes de malaxage, les divers autres ingrédients à l'exception du système de vulcanisation. On conduit alors un travail thermomécanique (phase non-productive) en une étape (durée totale du malaxage égale à environ 5 min), jusqu'à atteindre une température maximale de "tombée" d'environ 165°C. On récupère le mélange ainsi obtenu, on le refroidit puis on ajoute le système de vulcanisation (soufre et composé 1,2,4-triazine selon l'invention) sur un mélangeur externe (homo-finisseur) à 70°C, en mélangeant le tout (phase productive) pendant environ 5 à 6 min.

Les compositions ainsi obtenues sont ensuite calandrées soit sous la forme de plaques (épaisseur de 2 à 3 mm) ou de feuilles fines de caoutchouc pour la mesure de leurs propriétés physiques ou mécaniques, soit sous la forme de profilés utilisables directement, après découpage et/ou assemblage aux dimensions souhaitées, par exemple comme produits semi-finis pour pneumatiques, en particulier comme bandes de roulement de pneumatiques.

### III-3. Essais de caractérisation - Résultats

### A- Exemple 1

L'objet de cet exemple est de comparer les propriétés de compositions de caoutchouc comprenant du noir de carbone et de la silice, utilisable pour la fabrication d'une bande de roulement de pneumatique, comprenant la CBS ou le MBTS à titre d'accélérateur primaire de vulcanisation (composition C1 et C2), avec les propriétés de compositions de caoutchouc selon l'invention comprenant le composé C (composition C3), le composé A (composition C4) ou le composé B (composition C5) à titre d'accélérateur primaire de vulcanisation.

Les formulations des compositions sont données dans le tableau 1 ci-dessous. Les quantités sont exprimées en parties pour 100 parties en poids d'élastomère (pce).

**Tableau 1**

| Essais | C1 | C2 | C3 | C4 | C5 |
|---|---|---|---|---|---|
| BR (1) | 30 | 30 | 30 | 30 | 30 |
| SBR (2) | 70 | 70 | 70 | 70 | 70 |
| Noir de carbone (N234) (3) | 4 | 4 | 4 | 4 | 4 |
| Silice (4) | 80 | 80 | 80 | 80 | 80 |
| 6-PPD (5) | 2 | 2 | 2 | 2 | 2 |
| Cire ozone | | | | | |
| C32 ST (6) | 1,5 | 1,5 | 1,5 | 1,5 | 1,5 |
| Résine (7) | 20 | 20 | 20 | 20 | 20 |
| Silane liq (8) | 6,5 | 6,5 | 6,5 | 6,5 | 6,5 |
| SAD (9) | 2 | 2 | 2 | 2 | 2 |
| DPG (10) | 1,5 | 1,5 | 1,5 | 1,5 | 1,5 |
| ZnO | 1,5 | 1,5 | 1,5 | 1,5 | 1,5 |
| Soufre | 1,2 | 1,2 | 1,2 | 1,2 | 1,2 |
| CBS* | 1,9 | - | - | - | - |
| MBTS** | - | 1,2 | - | - | - |
| composé C | - | - | 2,0 | - | - |
| composé A | - | - | - | 1,9 | - |
| composé B | - | - | - | - | 1,4 |

| | | | | | |
|---|---|---|---|---|---|
| (1) polybutadiène avec 0,7% de 1-2 ; 1,7% de trans 1-4 ; 98% de cis 1-4 (Tg = -105°C) (% molaires) (2) copolymère butadiène-styrène SSBR (SBR préparé en solution) avec 25% de styrène, 59% de motifs polybutadiène 1-2 et 20% de motifs polybutadiène 1-4 trans (Tg = -24°C), (% molaires) ; taux exprimé en SBR sec (SBR étendu avec 9% en poids d'huile MES, soit un total de SSBR + huile égal à 76 pce) (3) noir de carbone (4) Silice "Zeosil 1165MP" de la société Rhodia, type "HDS" (BET et CTAB : environ 160 m2/g); (5) Agent anti-oxydant 6-para-phénylènediamine (6) Agent anti-ozonant (7) Résine aliphatique (pure C5) « Hikorez A=1100 » commercialisée par la société KOLON (8) agent de couplage (9) Stéarine « Pristeren » de la société Uniquema (10) Diphénylguanidine (Perkacit DPG de la société Flexsys) * CBS (Santocure CBS de la société Flexsys) ** MBTS de la société G- QUIMICA commercialisé sous la référence Rubator MBTS | | | | | |

Les propriétés rhéométriques à 150°C sont données dans le tableau 2 ci-dessous.

**Tableau 2**

| Essais | C1 | C2 | C3 | C4 | C5 |
|---|---|---|---|---|---|
| K | 1,03 | 0,59 | 0,21 | 0,18 | 0,56 |
| T0 (min) | 4,75 | 0,92 | 14,1 | 2,52 | 0,90 |

Les propriétés rhéométriques obtenues pour les compositions selon l'invention montrent que les composés C, A et B peuvent être utilisés comme accélérateurs de vulcanisation dans des compositions de caoutchouc comprenant une ou des charges renforçantes.

### B- Exemple 2

L'objet de cet exemple est de comparer les propriétés de compositions de caoutchouc comprenant du noir de carbone, utilisables pour la fabrication d'une bande de roulement de pneumatique, comprenant la CBS ou la MBTS à titre d'accélérateur primaire de vulcanisation (compositions C1 et C2), avec les propriétés de compositions de caoutchouc selon l'invention comprenant le composé C (composition C3), le composé D (composition C4) ou le composé B (composition C5) à titre d'accélérateur primaire de vulcanisation.

Les formulations des compositions sont données dans le tableau 3 ci-dessous. Les quantités sont exprimées en parties pour 100 parties en poids d'élastomère (pce).

**Tableau 3**

| Essais | C1 | C2 | C3 | C4 | C5 |
|---|---|---|---|---|---|
| NR (1) | 100 | 100 | 100 | 100 | 100 |
| Noir de carbone (N234) | 47 | 47 | 47 | 47 | 47 |
| SAD (2) | 2,5 | 2,5 | 2,5 | 2,5 | 2,5 |
| ZnO | 2,7 | 2,7 | 2,7 | 2,7 | 2,7 |
| Soufre | 1,5 | 1,5 | 1,5 | 1,5 | 1,5 |
| CBS* | 0,6 | - | - | - | - |
| MBTS** | - | 0,39 | - | - | - |
| composé C | - | - | 0,63 | - | - |
| composé D | - | - | - | 0,81 | - |
| composé B | - | - | - | - | 0,4 |

| | | | | | |
|---|---|---|---|---|---|
| (1) caoutchouc naturel (2) Stéarine « Pristeren » de la société Uniquema * CBS (Santocure CBS de la société Flexsys) ** MBTS de la société G- QUIMICA commercialisé sous la référence Rubator MBTS | | | | | |

Les propriétés rhéométriques à 150°C sont données dans le tableau 4 ci-dessous.

**Tableau 4**

| Essais | C1 | C2 | C3 | C4 | C5 |
|---|---|---|---|---|---|
| k | 0,98 | 0,32 | 0,10 | 0,09 | 0,42 |
| T0 (min) | 4,52 | 1,25 | 11,50 | 0,98 | 1,62 |

Les propriétés rhéométriques obtenues pour les compositions selon l'invention montrent que les composés C, D et B peuvent être utilisés comme accélérateurs de vulcanisation dans des compositions de caoutchouc comprenant une ou des charges renforçantes.

## Revendications

1. Composition de caoutchouc pour la fabrication de pneumatiques, à base d'un ou plusieurs élastomères diéniques, d'une ou plusieurs charges renforçantes et d'un système de vulcanisation, **caractérisée en ce que** ledit système de vulcanisation comprend un ou plusieurs composés 1,2,4-triazines choisis parmi les composés de formule 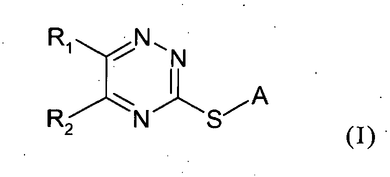 et 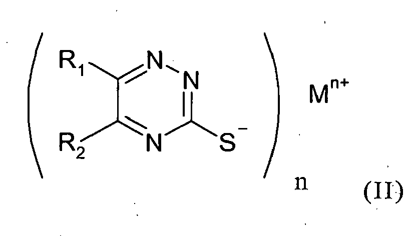 où
R₁ et R₂ représentent indépendamment H, un halogène, un groupe choisi par les groupes alcoxy et (C₁-C₁₀)alkylthio, ou un groupe hydrocarboné en C₁-C₂₅ choisi parmi les groupes alkyles linéaires, ramifiés ou cycliques, les groupes aralkyles, les groupes alkylaryles et les groupes aryles, et éventuellement interrompus par un ou plusieurs hétéroatomes, R₁ pouvant former un cycle avec R₂,
- A est choisi parmi
- -H,
- un groupe 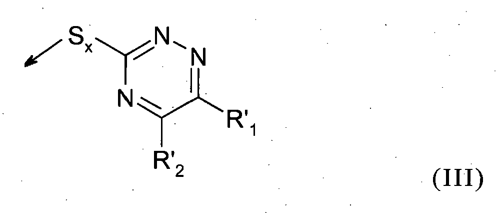 où
R'₁ est identique à R₁,
R'₂ est identique à R₂,
x est un entier supérieur ou égal à 1, préférentiellement inférieur ou égal à 10, plus préférentiellement inférieur ou égal à 8 et très préférentiellement inférieur ou égal à 6,
- un groupe 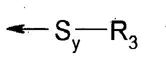
où R₃ représente un groupe hydrocarboné en C₁-C₁₈ éventuellement interrompu par un ou plusieurs hétéroatomes, de préférence un groupe CR'₃R₄R₅, où R'₃, R₄ et R₅ représentent indépendamment un groupe en C₁-C₁₇ éventuellement interrompu par un ou plusieurs hétéroatomes,
y est un entier supérieur ou égal à 1, préférentiellement inférieur ou égal à 10, plus préférentiellement inférieur ou égal à 8 et très préférentiellement inférieur ou égal à 6,
- un groupe 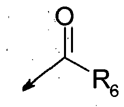
où R₆ est un groupe hydrocarboné en C₁-C₁₈, éventuellement interrompu par un ou plusieurs hétéroatomes,
- un groupe 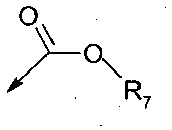
où R₇ est un groupe hydrocarboné en C₁-C₁₈, éventuellement interrompu par un ou plusieurs hétéroatomes,
- un groupe 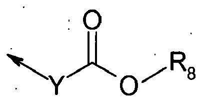
où Y est une chaîne hydrocarbonée en C₁-C₁₈, éventuellement interrompue ou substituée par un ou plusieurs hétéroatomes ; R₈ est un groupe hydrocarboné en C₁-C₁₈, éventuellement interrompu par un ou plusieurs hétéroatomes,
- un groupe 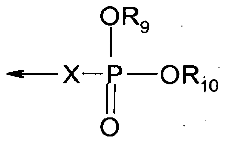
où X est une chaîne hydrocarbonée en C₁-C₁₈, éventuellement interrompue ou substituée par un ou plusieurs hétéroatomes ; R₉ et R₁₀ représentent indépendamment un groupe hydrocarboné en C₁-C₁₈, éventuellement interrompu par un ou plusieurs hétéroatomes, et
- un groupe 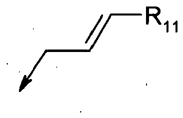
où R₁₁ est H ou un groupe hydrocarboné en C₁-C₁₈, éventuellement interrompu par un ou plusieurs hétéroatomes,
Mⁿ⁺ représente un cation métallique ou non métallique,
n représente un entier variant entre 1 et 4.

2. Composition selon la revendication 1, **caractérisée en ce que** R₁ et R₂ représentent indépendamment H ou un groupe phényle.

3. Composition selon la revendication 2, **caractérisée en ce que** R₂ représente un groupe phényle et R₁ représente H.

4. Composition selon l'une quelconque des revendications précédentes, **caractérisée en ce que** A est choisi parmi
- le groupe 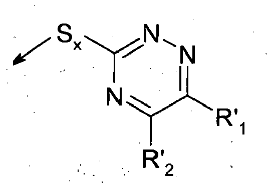
- le groupe 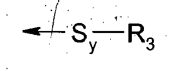
- le groupe 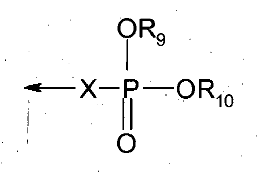
- le groupe 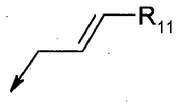
où x, R'₁, R'₂, y, R₃, X, R₉, R₁₀ et R₁₁ sont tels que définis dans la revendication 1.

5. Composition selon l'une quelconque des revendications précédentes, **caractérisée en ce que** les groupes R₃ et R₆ à R₁₁ sont choisis indépendamment parmi les groupes alkyles linéaires, ramifiés ou cycliques, les groupes aryles, les groupes aralkyles et les groupes alkylaryles.

6. Composition selon l'une quelconque des revendications précédentes, **caractérisée en ce que** les groupes X et Y sont choisis indépendamment parmi les groupes alkylènes linéaires, ramifiés ou cycliques, les groupes arylènes et les groupes alkylarylènes.

7. Composition selon l'une quelconque des revendications précédentes, **caractérisée en ce que** le ou lesdits composés 1,2,4-triazines représentent de 0,1 à 7 pce, de préférence de 0,2 à 7 pce, de préférence encore de 0,5 à 7 pce, mieux de 0,5 à 5 pce (parties en poids pour cent d'élastomère diénique).

8. Procédé pour préparer une composition de caoutchouc pour la fabrication de pneumatiques telle que définie à l'une quelconque des revendications précédentes **caractérisé en ce qu'**il comprend les étapes suivantes :
- incorporer à ou auxdits élastomères diéniques, au cours d'une première étape, la ou les charges renforçantes, en malaxant thermomécaniquement le tout, en une ou plusieurs fois, jusqu'à atteindre une température maximale comprise entre 110°C et 190°C ;
- incorporer ensuite, au cours d'une seconde étape, le système de réticulation et malaxer le tout jusqu'à une température maximale inférieure à 110°C.

9. Pneumatique comprenant une composition de caoutchouc telle que définie à l'une quelconque des revendications 1 à 7.

10. Utilisation comme accélérateur de vulcanisation dans une composition à base d'un ou plusieurs élastomères diéniques, d'une ou plusieurs charges renforçantes et d'un système de vulcanisation, d'un ou plusieurs composés 1,2,4-triazines de formule (I) et/ou (II) tels que définis dans l'une quelconque des revendications 1 à 6.

11. 1,2,4-Triazine de formule 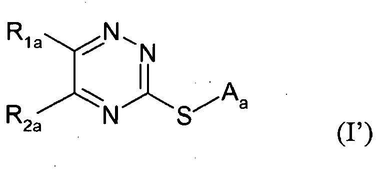 où
R₁ₐ et R₂ₐ représentent indépendamment H, un halogène, un groupe choisi par les groupes alcoxy et (C₁-C₁₀)alkylthio, ou un groupe hydrocarboné en C₁-C₂₅ choisi parmi les groupes alkyles linéaires, ramifiés ou cycliques, les groupes aralkyles, les groupes alkylaryles et les groupes aryles, et éventuellement interrompus par un ou plusieurs hétéroatomes, R₁ₐ pouvant former un cycle avec R₂ₐ,
- Aₐ est choisi parmi
- un groupe 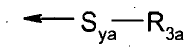
où R₃ₐ représente un groupe hydrocarboné en C₁-C₁₈, éventuellement interrompu par un ou plusieurs hétéroatomes, à la condition que R₃ₐ ne comporte pas de 1,2,4-triazine, et à la condition que le composé (I') ne soit pas de formule 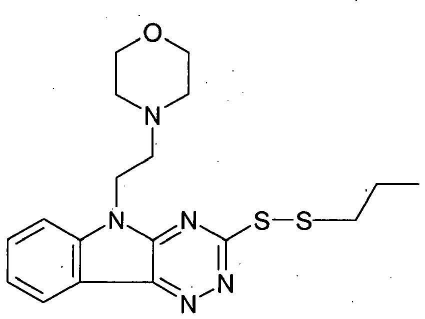 yₐ est un entier supérieur ou égal à 1, préférentiellement inférieur ou égal à 10, plus préférentiellement inférieur ou égal à 8 et très préférentiellement inférieur ou égal à 6,
- un groupe 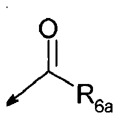
où R₆ₐ est un groupe hydrocarboné en C₁-C₁₈, éventuellement interrompu par un ou plusieurs hétéroatomes, étant entendu que R₆ₐ est différent d'un groupe phényle éventuellement substitué, et étant entendu que si R₆ₐ représente un groupe méthyle, R₁ₐ et R₂ₐ ne forment pas de cycle et R₁ₐ et R₂ₐ ne représentent pas respectivement un méthyle et un hydroxyle,
- un groupe 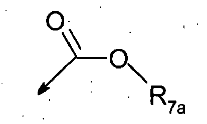
où R₇ₐ est un groupe hydrocarboné en C₁-C₁₈, éventuellement interrompu par un ou plusieurs hétéroatomes, étant entendu que si R₇ₐ représente un groupe éthyle, R₁ₐ et R₂ₐ ne forment pas de cycle,
- un groupe 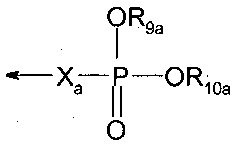
où Xₐ est une chaîne hydrocarbonée en C₁-C₁₈, éventuellement interrompue ou substituée par un ou plusieurs hétéroatomes ;
R₉ₐ et R₁₀ₐ représentent indépendamment un groupe hydrocarboné en C₁-C₁₈, éventuellement interrompu par un ou plusieurs hétéroatomes.

12. 1,2,4-Triazine selon la revendication 11, **caractérisée en ce que** R₁ₐ et R₂ₐ représentent indépendamment H ou un groupe phényle.

13. 1,2,4-Triazine selon la revendication 11 à 12, **caractérisée en ce que** Aa est choisi parmi :
- le groupe 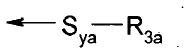
- le groupe 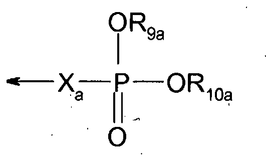
où yₐ, R₃ₐ, Xₐ, R₉ₐ et R₁₀ₐ sont tels que définis à la revendication 11.

14. 1,2,4-Triazine selon l'une quelconque des revendications 11 à 13, **caractérisée en ce que** les groupes R₃ₐ, R₆ₐ, R₇ₐ et R₉ₐ à R₁₀ₐ sont choisis indépendamment parmi les groupes alkyles linéaires, ramifiés ou cycliques, les groupes aryles, les groupes aralkyles et les groupes alkylaryles.

15. 1,2,4-Triazine selon l'une quelconque des revendications 11 à 14, **caractérisée en ce que** le groupe Xₐ est choisi parmi les groupes alkylènes linéaires, ramifiés ou cycliques, les groupes arylènes et les groupes alkylarylènes.

## Patentansprüche

1. Kautschukzusammensetzung zur Herstellung von Reifen auf Basis eines oder mehrerer Dienelastomere, eines oder mehrerer verstärkender Füllstoffe und eines Vulkanisationssystems, **dadurch gekennzeichnet, dass** das Vulkanisationssystem eine oder mehrere 1,2,4-Triazinverbindungen umfasst, die aus den Verbindungen der Formel 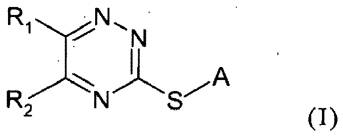 und 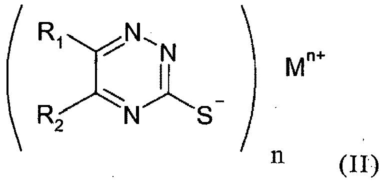 ausgewählt sind, wobei
R₁ und R₂ unabhängig für H, Halogen, eine aus Alkoxy- und (C₁-C₁₀)-Alkylthiogruppen ausgewählte Gruppe oder eine C₁-C₂₅-Kohlenwasserstoffgruppe, die aus linearen, verzweigten oder cyclischen Alkylgruppen, Aralkylgruppen, Alkylarylgruppen und Arylgruppen, die gegebenenfalls durch ein oder mehrere Heteroatome unterbrochen sind, ausgewählt ist, stehen, wobei R₁ mit R₂ einen Ring bilden kann,
- A aus
- -H,
- einer Gruppe 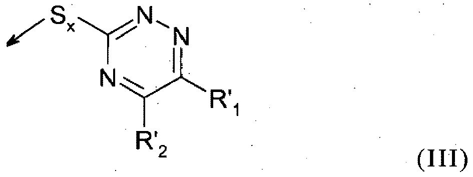 wobei
R'₁ mit R₁ identisch ist,
R'₂ mit R₂ identisch ist,
x für eine ganze Zahl größer gleich 1, vorzugsweise kleiner gleich 10, weiter bevorzugt kleiner gleich 8 und ganz besonders bevorzugt kleiner gleich 6 steht,
- einer Gruppe 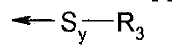 wobei R₃ für eine C₁-C₁₈-Kohlenwasserstoffgruppe, die gegebenenfalls durch ein oder mehrere Heteroatome unterbrochen ist, vorzugsweise eine Gruppe CR'₃R₄R₅, wobei R'₃, R₄ und R₅ unabhängig für eine C₁-C₁₇-Gruppe, die gegebenenfalls durch ein oder mehrere Heteroatome unterbrochen ist, stehen, steht, y für eine ganze Zahl größer gleich 1, vorzugsweise kleiner gleich 10, weiter bevorzugt kleiner gleich 8 und ganz besonders bevorzugt kleiner gleich 6 steht,
- einer Gruppe 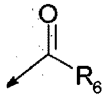 wobei R₆ für eine C₁-C₁₈-Kohlenwasserstoffgruppe, die gegebenenfalls durch ein oder mehrere Heteroatome unterbrochen ist, steht,
- einer Gruppe 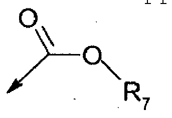 wobei R₇ für eine C₁-C₁₈-Kohlenwasserstoffgruppe, die gegebenenfalls durch ein oder mehrere Heteroatome unterbrochen ist, steht,
- einer Gruppe 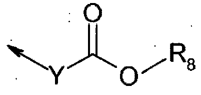 wobei Y für eine C₁-C₁₈-Kohlenwasserstoffkette, die gegebenenfalls durch ein oder mehrere Heteroatome unterbrochen oder substituiert ist, steht,
R₈ für eine C₁-C₁₈-Kohlenwasserstoffgruppe, die gegebenenfalls durch ein oder mehrere Heteroatome unterbrochen ist, steht,
- einer Gruppe 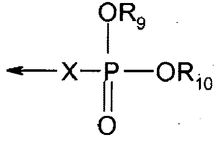 wobei X für eine C₁-C₁₈-Kohlenwasserstoffkette, die gegebenenfalls durch ein oder mehrere Heteroatome unterbrochen oder substituiert ist, steht,
R₉ und R₁₀ unabhängig für eine C₁-C₁₈-Kohlenwasserstoffgruppe, die gegebenenfalls durch ein oder mehrere Heteroatome unterbrochen ist, stehen, und
- einer Gruppe 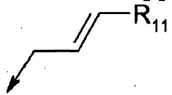 wobei R₁₁ für H oder eine C₁-C₁₈-Kohlenwasserstoffgruppe, die gegebenenfalls durch ein oder mehrere Heteroatome unterbrochen ist, steht,
ausgewählt ist,
Mⁿ⁺ für ein Metall- oder Nichtmetallkation steht,
n für eine ganze Zahl zwischen 1 und 4 steht.

2. Zusammensetzung nach Anspruch 1, **dadurch gekennzeichnet, dass** R₁ und R₂ unabhängig für H oder eine Phenylgruppe stehen.

3. Zusammensetzung nach Anspruch 2, **dadurch gekennzeichnet, dass** R₂ für eine Phenylgruppe steht und R₁ für H steht.

4. Zusammensetzung nach einem der vorhergehenden Ansprüche, **dadurch gekennzeichnet, dass** A aus
- der Gruppe 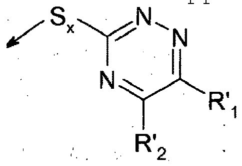
- der Gruppe 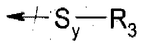
- der Gruppe 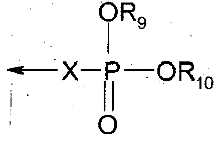
- der Gruppe 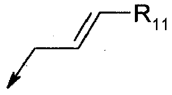
ausgewählt ist, wobei x, R'₁, R'₂, y, R₃, X, R₉, R₁₀ und R₁₁ wie in Anspruch 1 definiert sind.

5. Zusammensetzung nach einem der vorhergehenden Ansprüche, **dadurch gekennzeichnet, dass** die Gruppen R₃ und R₆ bis R₁₁ unabhängig aus linearen, verzweigten oder cyclischen Alkylgruppen, Arylgruppen, Aralkylgruppen und Alkylarylgruppen ausgewählt sind.

6. Zusammensetztung nach einem der vorhergehenden Ansprüche, **dadurch gekennzeichnet, dass** die Gruppen X und Y unabhängig aus linearen, verzweigten oder cyclischen Alkylengruppen, Arylengruppen und Alkylarylengruppen ausgewählt sind.

7. Zusammensetzung nach einem der vorhergehenden Ansprüche, **dadurch gekennzeichnet, dass** die 1,2,4-Triazinverbindung bzw. die 1,2,4-Triazinverbindungen 0,1 bis 7 phe, vorzugsweise 0,2 bis 7 phe, noch weiter bevorzugt 0,5 bis 7 phe und noch besser 0,5 bis 5 phe (Gewichtsteile pro hundert Teile Dienelastomer) ausmacht bzw. ausmachen.

8. Verfahren zur Herstellung einer Kautschukzusammensetzung zur Herstellung von Reifen gemäß einem der vorhergehenden Ansprüche, **dadurch gekennzeichnet, dass** man:
- im Lauf eines ersten Schritts den verstärkenden Füllstoff bzw. die verstärkenden Füllstoffe in das Dienelastomer bzw. die Dienelastomere einarbeitet, indem man das Ganze ein- oder mehrmals thermomechanisch knetet, bis eine Höchsttemperatur zwischen 110°C und 190°C erreicht ist;
- dann im Lauf eines zweiten Schritts das Vernetzungssystem einarbeitet und das Ganze bis zu einer Höchsttemperatur von weniger als 110°C knetet.

9. Reifen, umfassend eine Kautschukzusammensetzung gemäß einem der Ansprüche 1 bis 7.

10. Verwendung einer oder mehrerer 1,2,4-Triazinverbindungen der Formel (I) und/oder (II) gemäß einem der Ansprüche 1 bis 6 als Vulkanisationsbeschleuniger in einer Zusammensetzung auf der Basis eines oder mehrerer Dienelastomere, eines oder mehrerer verstärkender Füllstoffe und eines Vulkanisationssystems.

11. 1,2,4-Triazin der Formel 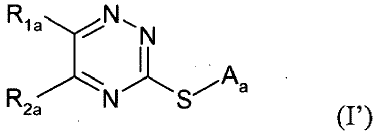 wobei
R₁ₐ und R₂ₐ unabhängig für H, Halogen, eine aus Alkoxy- und (C₁-C₁₀)-Alkylthiogruppen ausgewählte Gruppe oder eine C₁-C₂₅-Kohlenwasserstoffgruppe, die aus linearen, verzweigten oder cyclischen Alkylgruppen, Aralkylgruppen, Alkylarylgruppen und Arylgruppen, die gegebenenfalls durch ein oder mehrere Heteroatome unterbrochen sind, ausgewählt ist, stehen, wobei R₁ₐ mit R₂ₐ einen Ring bilden kann,
- Aₐ aus
- einer Gruppe 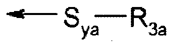 wobei R₃ₐ für eine C₁-C₁₈-Kohlenwasserstoffgruppe, die gegebenenfalls durch ein oder mehrere Heteroatome unterbrochen ist, steht, mit der Maßgabe, dass R₃ₐ kein 1,2,4-Triazin umfasst, und mit der Maßgabe, dass die Verbindung der Formel (I') nicht die Formel 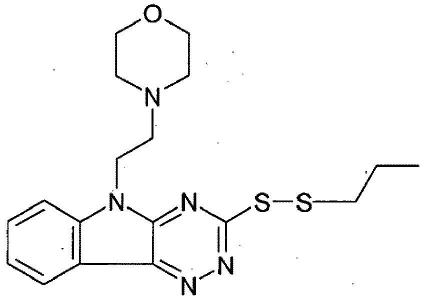 aufweist,
yₐ für eine ganze Zahl größer gleich 1, vorzugsweise kleiner gleich 10, weiter bevorzugt kleiner gleich 8 und ganz besonders bevorzugt kleiner gleich 6 steht,
- einer Gruppe 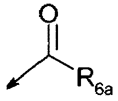 wobei R₆ₐ für eine C₁-C₁₈-Kohlenwasserstoffgruppe, die gegebenenfalls durch ein oder mehrere Heteroatome unterbrochen ist, steht, wobei es sich versteht, dass R₆ₐ von einer gegebenenfalls substituierten Phenylgruppe verschieden ist, und wobei es sich versteht, dass dann, wenn R₆ₐ für eine Methylgruppe steht, R₁ₐ und R₂ₐ keinen Ring bilden und R₁ₐ und R₂ₐ respektive nicht für ein Methyl und ein Hydroxyl stehen,
- einer Gruppe 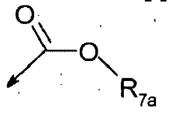 wobei R₇ₐ für eine C₁-C₁₈-Kohlenwasserstoffgruppe, die gegebenenfalls durch ein oder mehrere Heteroatome unterbrochen ist, steht, wobei es sich versteht, dass dann, wenn R₇ₐ für eine Ethylgruppe steht, R₁ₐ und R₂ₐ keinen Ring bilden,
- einer Gruppe 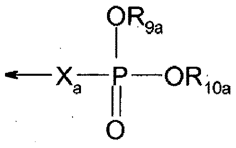 wobei Xₐ für eine C₁-C₁₈-Kohlenwasserstoffkette, die gegebenenfalls durch ein oder mehrere Heteroatome unterbrochen oder substituiert ist, steht,
R₉ₐ und R₁₀ₐ unabhängig für eine C₁-C₁₈-Kohlenwasserstoffgruppe, die gegebenenfalls durch ein oder mehrere Heteroatome unterbrochen ist, stehen,
ausgewählt ist.

12. 1,2,4-Triazin nach Anspruch 11, **dadurch gekennzeichnet, dass** R₁ₐ und R₂ₐ unabhängig für H oder eine Phenylgruppe stehen.

13. 1,2,4-Triazin nach Anspruch 11 oder 12, **dadurch gekennzeichnet, dass** Aₐ aus
- der Gruppe 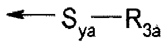
- der Gruppe 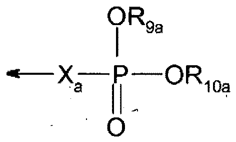
ausgewählt ist, wobei yₐ, R₃ₐ, Xₐ, R₉ₐ und R₁₀ₐ wie in Anspruch 11 definiert sind.

14. 1,2,4-Triazin nach einem der Ansprüche 11 bis 13, **dadurch gekennzeichnet, dass** die Gruppen R₃ₐ, R₆ₐ, R₇ₐ und R₉ₐ bis R₁₀ₐ unabhängig aus linearen, verzweigten oder cyclischen Alkylgruppen, Arylgruppen, Aralkylgruppen und Alkylarylgruppen ausgewählt sind.

15. 1,2,4-Triazin nach einem der Ansprüche 11 bis 14, **dadurch gekennzeichnet, dass** die Gruppe Xₐ aus linearen, verzweigten oder cyclischen Alkylengruppen, Arylengruppen und Alkylarylengruppen ausgewählt ist.

## Claims

1. Rubber composition for the manufacture of tyres, based on one or more diene elastomers, on one or more reinforcing fillers and on a vulcanization system, **characterized in that** the said vulcanization system comprises one or more 1,2,4-triazine compounds chosen from the compounds of formula 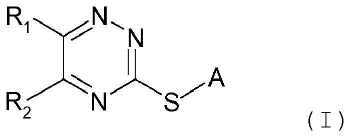 and 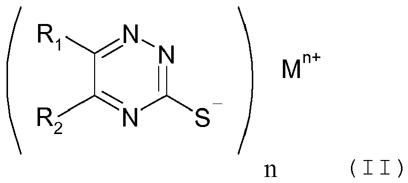 where:
R₁ and R₂ independently represent H, a halogen, a group chosen from alkoxy and (C₁-C₁₀)alkylthio groups, or a C₁-C₂₅ hydrocarbon group chosen from linear, branched or cyclic alkyl groups, aralkyl groups, alkylaryl groups and aryl groups, and optionally interrupted by one or more heteroatoms, it being possible for R₁ to form a ring with R₂,
- A is chosen from
-H,
a group 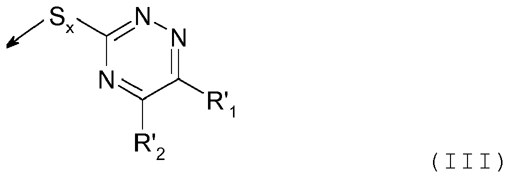 where:
R'₁ is identical to R₁,
R'₂ is identical to R₂,
x is an integer greater than or equal to 1, preferably less than or equal to 10, more preferably less than or equal to 8 and very preferably less than or equal to 6,
a group 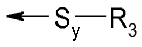 where R₃ represents a C₁-C₁₈ hydrocarbon group optionally interrupted by one or more heteroatoms, preferably a CR'₃R₄R₅ group, where R'₃, R₄ and R₅ independently represent a C₁-C₁₇ group optionally interrupted by one or more heteroatoms,
y is an integer greater than or equal to 1, preferably less than or equal to 10, more preferably less than or equal to 8 and very preferably less than or equal to 6,
a group 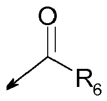 where R₆ is a C₁-C₁₈ hydrocarbon group, optionally interrupted by one or more heteroatoms,
a group 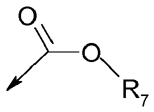 where R₇ is a C₁-C₁₈ hydrocarbon group, optionally interrupted by one or more heteroatoms,
a group 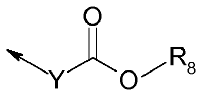 where Y is a C₁-C₁₈ hydrocarbon chain, optionally interrupted or substituted by one or more heteroatoms;
R₈ is a C₁-C₁₈ hydrocarbon group, optionally interrupted by one or more heteroatoms,
a group 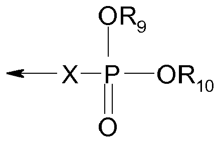 where X is a C₁-C₁₈ hydrocarbon chain, optionally interrupted or substituted by one or more heteroatoms;
R₉ and R₁₀ independently represent a C₁-C₁₈ hydrocarbon group, optionally interrupted by one or more heteroatoms, and
a group 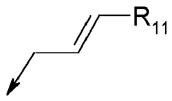 where R₁₁ is H or a C₁-C₁₈ hydrocarbon group, optionally interrupted by one or more heteroatoms,
Mⁿ⁺ represents a metal or non-metal cation,
n represents an integer varying between 1 and 4.

2. Composition according to Claim 1, **characterized in that** R₁ and R₂ independently represent H or a phenyl group.

3. Composition according to Claim 2, **characterized in that** R₂ represents a phenyl group and R₁ represents H.

4. Composition according to any one of the preceding claims, **characterized in that** A is chosen from
the group 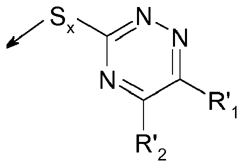 the group 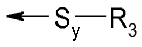 the group 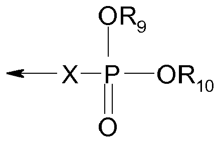 the group 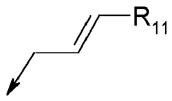 where x, R'₁, R'₂, y, R₃, X, R₉, R₁₀ and R₁₁ are as defined in Claim 1.

5. Composition according to any one of the preceding claims, **characterized in that** the R₃ and R₆ to R₁₁ groups are independently chosen from linear, branched or cyclic alkyl groups, aryl groups, aralkyl groups and alkylaryl groups.

6. Composition according to any one of the preceding claims, **characterized in that** the X and Y groups are independently chosen from linear, branched or cyclic alkylene groups, arylene groups and alkylarylene groups.

7. Composition according to any one of the preceding claims, **characterized in that** the said 1,2,4-triazine compound or compounds represent from 0.1 to 7 phr, preferably from 0.2 to 7 phr, more preferably from 0.5 to 7 phr and better still from 0.5 to 5 phr (parts by weight per hundred of diene elastomer).

8. Process for preparing a rubber composition for the manufacture of tyres as defined in any one of the preceding claims, **characterized in that** it comprises the following stages:
- incorporating the reinforcing filler or fillers in the said diene elastomer or elastomers during a first stage, everything being kneaded thermomechanically, in one or more goes, until a maximum temperature of between 110°C and 190°C is reached;
- subsequently incorporating, during a second stage, the crosslinking system and kneading everything up to a maximum temperature of less than 110°C.

9. Tyre comprising a rubber composition as defined in any one of Claims 1 to 7.

10. Use, as vulcanization accelerator in a composition based on one or more diene elastomers, on one or more reinforcing fillers and on a vulcanization system, of one or more 1,2,4-triazine compounds of formula (I) and/or (II) as defined in any one of Claims 1 to 6.

11. 1,2,4-Triazine of formula 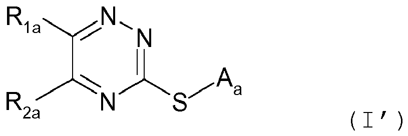 where:
R₁ₐ and R₂ₐ independently represent H, a halogen, a group chosen from alkoxy and (C₁-C₁₀)alkylthio groups, or a C₁-C₂₅ hydrocarbon group chosen from linear, branched or cyclic alkyl groups, aralkyl groups, alkylaryl groups and aryl groups, and optionally interrupted by one or more heteroatoms, it being possible for R₁ₐ to form a ring with R₂ₐ,
- Aₐ is chosen from
- a group 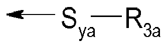 where R₃ₐ represents a C₁-C₁₈ hydrocarbon group, optionally interrupted by one or more heteroatoms, provided that R₃ₐ does not comprise 1,2,4-triazine and provided that the compound (I') is not of formula 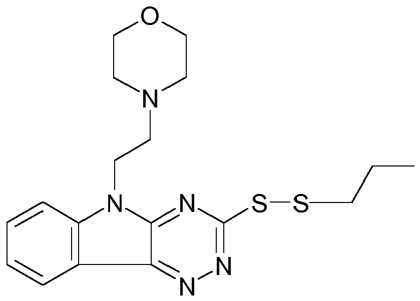 yₐ is an integer greater than or equal to 1, preferably less than or equal to 10, more preferably less than or equal to 8 and very preferably less than or equal to 6,
- a group 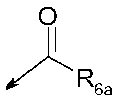 where R₆ₐ is a C₁-C₁₈ hydrocarbon group, optionally interrupted by one or more heteroatoms, it being understood that R₆ₐ is other than an optionally substituted phenyl group and it being understood that, if R₆ₐ represents a methyl group, R₁ₐ and R₂ₐ do not form a ring and R₁ₐ and R₂ₐ respectively do not represent a methyl and a hydroxyl,
- a group 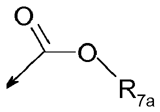 where R₇ₐ is a C₁-C₁₈ hydrocarbon group, optionally interrupted by one or more heteroatoms, it being understood that, if R₇ₐ represents an ethyl group, R₁ₐ and R₂ₐ do not form a ring,
- a group 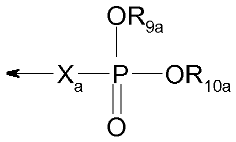 where Xₐ is a C₁-C₁₈ hydrocarbon chain, optionally interrupted or substituted by one or more heteroatoms;
R₉ₐ and R₁₀ₐ independently represent a C₁-C₁₈ hydrocarbon group, optionally interrupted by one or more heteroatoms.

12. 1,2,4-Triazine according to Claim 11, **characterized in that** R₁ₐ and R₂ₐ independently represent H or a phenyl group.

13. 1,2,4-Triazine according to Claim 11 or 12, **characterized in that** Aa is chosen from:
- the group 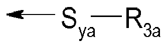
- the group 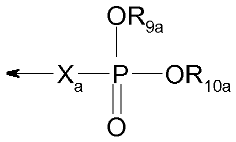
where yₐ, R₃ₐ, Xₐ, R₉ₐ and R₁₀ₐ are as defined in Claim 11.

14. 1,2,4-Triazine according to any one of Claims 11 to 13, **characterized in that** the R₃ₐ, R₆ₐ, R₇ₐ and R₉ₐ to R₁₀ₐ groups are independently chosen from linear, branched or cyclic alkyl groups, aryl groups, aralkyl groups and alkylaryl groups.

15. 1,2,4-Triazine according to any one of Claims 11 to 14, **characterized in that** the Xₐ group is chosen from linear, branched or cyclic alkylene groups, arylene groups and alkylarylene groups.
